# EUROPEAN PATENT APPLICATION

(11) **EP 4 170 039 A1**
(43) Date of publication of application: **26.04.2023**
(21) Application number: 21386064.6
(22) Date of filing: 22.10.2021
(51) Int. Cl.: C12N 15/82, C07K 14/16

(54) **MODIFYING THE IMMUNE RESPONSE IN PLANTS**

(71) Applicant: The Sainsbury Laboratory, Norwich, Norfolk NR4 7UH (GB)
(72) Inventor: Kourelis, Jiorgos, Norwich, NR1 1NS (GB); Marchal, Clemence, Norwich, NR47HZ (GB); Kamoun, Sophien, Norwich, NR1 3LY (GB)
(74) Representative: Marks & Clerk LLP

(57) **Abstract**

Chimeric proteins comprising a binding molecule, preferably a single chain antibody, linked to a plant immune receptor protein, are disclosed.

## Description

### Field of the invention

The present invention relates to methods and compositions for modifying the immune response in plants.

### Background to the invention

Each year, staple crops around the world suffer massive losses in yield owing to the destructive effects of pathogens. Improving the disease resistance of crops by boosting their immunity has been a key objective of agricultural biotechnology ever since the discovery of plant immune receptors in the 1990s. Plants lack an adaptive immune system and rely on innate immune receptors to detect invading pathogens and pests. One component of this innate immune system includes nucleotide-binding leucine-rich repeat (NLR) proteins that detect effector proteins secreted by pathogens either by directly binding the effector proteins or by indirectly binding the effector proteins via effector-targeted host proteins. Other classes of immune receptors that detect pathogen molecules include receptor kinases (RKs) and receptor proteins (RPs). However, efforts to re-tool NLR and other plant immune receptor proteins to design new-to-nature activities have been limited to modifying natural components for instance through receptor mutagenesis or domain shuffling.

A subset of NLR immune receptors carry unconventional integrated domains. These receptors carry a "decoy" domain that mimics the natural target of a pathogen effector. This decoy domain binds pathogen effectors and activates an immune response. For example, the rice NLR Pik-1 carries an integrated heavy metal associated (HMA) domain between its N-terminal coiled coil (CC) domain and the central nucleotide binding adaptor domain (NB-ARC) (Bialas *et al.,* 2018), which binds secreted protein effectors from the blast fungus. Activated Pik-1 relies on its partner Pik-2, which belongs to the MADA type NLRs that are thought to be activated like a canonical plant NLR called ZAR1. The integrated HMA domain of Pik-1 can be mutated to confer new pathogen effector responses. However, to date this has only been effective for effectors from the blast fungus.

Bialas et al. 2018 ("Lessons in effector and NLR biology of plant-microbe systems", MPMI, 2018, 31(1), p.34-35) discusses lessons in pathogen effector and NLR biology that have emerged from studying the effectors AVR-Pik, AVR-Pia and AVR-Pii, as well as their matching NLR receptors, and which are broadly applicable to other plant-microbe systems.

WO 2019/108619 discloses engineered NLRs which are capable of evading suppression of cell death caused by pathogen effectors. The amino acid sequence of an NLR is modified by replacing one or more regions or blocks of multiple contiguous amino acids in one or more of its domains with the corresponding regions or blocks of contiguous amino acids in the same domain(s) of at least one other NLR protein.

There is a need to improve the disease resistance of crops, and to provide crop resistance to a range of pathogens to mitigate recurrent cycles of plant disease outbreaks.

### Summary of Invention

The present invention concerns the fusion of one or more binding molecules, preferably nanobodies, with a plant immune receptor to produce functional disease resistance genes with new-to-nature functionalities. The fusion proteins of the invention, referred to herein as "Pikobodies", have the potential to yield resistance against any pathogen or pest that delivers effectors inside host plant cells. As mammalian adaptive immunity has the capacity to generate antibodies against virtually any antigen it is exposed to, harnessing nanobodies for plant immunity would potentially enable engineered receptors that respond to any plant pathogen molecule. Thus, the Pikobodies of the present invention can be used for providing a pseudo-adaptive immune system to plants, supplementing the innate immune system.

Thus, in a first aspect of the invention, there is provided a chimeric protein comprising a binding molecule, preferably a nanobody, linked to a plant immune receptor protein. Preferably the nanobody is specific for an effector protein from a plant pathogen, and the chimeric protein is capable of activating immune signalling in a plant cell upon binding of the effector protein to the nanobody. However, in certain embodiments, the nanobody may be specific for other ligands; for example, the nanobody may be specific for a label such as GFP which is attached to a test molecule, such that the system can be used for monitoring activity of that test molecule; and/or the chimeric protein may be used as a sensor to detect presence of a target molecule within a cell.

Nanobodies are also known as single-domain antibodies (sdAb), and are antibody fragments consisting of a single monomeric variable domain of an antibody. Typically this is derived from a camelid single-chain antibody VHH region, although chondrichthyan VNAR fragments are also used. Phage display libraries have been generated from, for example, nurse sharks. Alternatively, it is possible to produce sdAbs from murine or other antibodies which typically have both heavy and light chains; or from mammals engineered to produce heavy-chain only antibodies. The present invention is intended to encompass use of sdAbs from any suitable source. Databases of existing sdAbs are available, for example, http://www.sdab-db.ca/, described in ACS Synth. Biol. 2018, 7, 11, 2480-2484. The terms "nanobodies" and "sdAbs" are used herein interchangeably. Given that mammalian adaptive immunity has the capacity to generate antibodies against virtually any antigen it is exposed to, harnessing antibodies for plant immunity would potentially enable engineering receptors that respond to any plant pathogen molecule. Camelid VHHs are particularly suitable because antigen binding affinity is coded by a small 10-15 kD domain that is soluble and has many useful properties in biotechnological applications.

In embodiments of the invention, the binding molecule is a nanobody; however, the present inventors believe that other binding molecules may also be suitable for use in the invention. For example, the binding molecule may preferably be one containing an immunoglobulin domain which recognises a particular target. Examples of such molecules can include any molecule that bind to target proteins with affinity. This can include molecules derived from plant or microbe proteins and that are used to mimic antibody binding affinities, such as DARPins, Monobodies, Affibodies, Affimers, as well as pathogen effectors and the host targets of effectors. Relevant references for such molecules include "Non-immunoglobulin scaffolds: a focus on their targets" https://doi.org/10.1016/j.tibtech.2015.03.012; and "Affimer proteins are versatile and renewable affinity reagents" https://elifesciences.org/articles/24903 .

The nanobody may be specific for a plant pathogen, a parasite or pest or a parasitic plant effector protein. In embodiments, this may be any secreted protein from bacteria, fungi, oomycetes, viruses, nematodes, insects, parasitic plants. The target proteins can be Oomycete effectors (e.g. RXLR, RXLR-WY/LWY, Crinkler (CRN)), MAX effectors of Magnaporthe spp., RALPH Effectors of Powdery Mildew, effectors of the rust fungi etc. Examples of filamentous plant pathogens can be found in Franceschetti M, Maqbool A, Jiménez Dalmaroni MJ, Pennington HG, Kamoun S, Banfield MJ. 2017. Effectors of filamentous plant pathogens: commonalities amid diversity. Microbiol Mol Biol Rev 81:e00066-16. https://doi.org/10.1128/MMBR.00066-16. Of course, it will be appreciated that, given the versatility of the described system, this should not be understood as being limited to any specific source of protein.

All classes of plant immune receptors are modular/multidomain proteins that perceive pathogen-derived molecules (effectors) either directly or indirectly. The plant immune system includes intracellular NLRs as discussed above, and membrane-anchored pattern recognition receptors which includes receptor kinases (RKs) and receptor proteins (RPs). RKs and RPs contain a variable ectodomain that usually functions to recognize either conserved microbial signatures known as pathogen-associated molecular patterns or damage indicators known as danger-associated molecular patterns. In the direct model, the receptor protein binds a pathogen effector (non-self recognition). In the indirect model, the receptor recognizes modifications of additional host protein(s) targeted by the effector. Such intermediate host protein(s) can be effector targets, guardees or decoys (modified self recognition). When activated after binding to non-self or modified self molecules, all classes of plant receptors undergo major conformational changes that rearrange their intra -and inter-molecular domain interactions. Because all plant receptors have a modular architecture and undergo domain interaction restructuring after activation, and because of the proof-of-concept disclosed herein relating to NLR-nanobody fusions, we believe it is plausible to create new-to-nature fusions of any NLR, RK or RP with a nanobody. In addition, RPs and RKs are known to require NLR proteins to activate an effective immune response, thus new-to-nature NLRs can also modulate the activity of pattern recognition receptors. Binding of non-self or modified self molecules to the nanobody perturb intra- and inter-molecular domain structure and therefore release the activate receptor for immune signaling.

The plant immune receptor is preferably an NLR protein. Plant NLR proteins usually contain a C-terminal LRR domain and a central NB-ARC domain, and generally fall into one of two groups, depending on their N-terminal structures, CNL (CC-NB-LRR) with an N-terminal coiled-coil domain and TNL (TIR-NB-LRR) with an N-terminal Toll/interleukin-1 receptor domain (TIR). The NLR is preferably of the CC-NB-LRR type.

The NLR may be derived from a wild-type NLR protein, preferably one having an integrated decoy domain. Typically the wild-type NLR will be of the structure CC-NB-LRR, and more specifically CC-decoy-NB-LRR. By "derived from" is meant that the NLR portion of the chimeric protein may be based on the sequence and/or structure of the wild-type NLR; however, the NLR portion need not be directly obtained from a wild-type NLR protein - that is, the NLR portion need not physically come from a plant. In embodiments, "derived from" may mean that the wild-type NLR and the NLR portion of the chimeric protein share at least 60%, 70%, 75%, 80%, 85%, 86%, 87%, 88%, 89%, 90%, 91%, 92%, 93%, 94%, 95%, 96%, 97%, 98%, or 99% sequence homology between the common regions. This may be compared across amino acid sequences, or across nucleic acid sequences encoding for the proteins. For example, where the chimeric protein is derived from a CC-decoy-NB-LRR NLR, and has the structure CC-nanobody-NB-LRR, then the two proteins may be at least 60%, 70%, 75%, 80%, 85%, 86%, 87%, 88%, 89%, 90%, 91%, 92%, 93%, 94%, 95%, 96%, 97%, 98%, or 99% identical over the CC-NB-LRR domains.

The NLR portion of the chimeric protein may be a homolog, ortholog, or functional variant of an NLR protein. The term "functional variant" as used herein with reference to any of the sequences described herein refers to a variant gene or amino acid sequence or part of the gene or amino acid sequence that retains the biological function of the full non-variant sequence. A functional variant also comprises a variant of the gene of interest that has sequence alterations that do not affect function, for example in non- conserved residues. Also encompassed is a variant that is substantially identical, i.e. has only some sequence variations, for example in non-conserved residues, compared to the wild type sequences as shown herein and is biologically active. Alterations in a nucleic acid sequence which result in the production of a different amino acid at a given site that do not affect the functional properties of the encoded polypeptide are well known in the art. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the polypeptide molecule would also not be expected to alter the activity of the polypeptide. Each of the proposed modifications is well within the routine skill in the art, as is determination of retention of biological activity of the encoded products.

In one embodiment, a functional variant has at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99% overall sequence identity to the non-variant nucleic acid or amino acid sequence.

The term homolog, as used herein, also designates a gene ortholog from other plant species. A homolog may have, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99% overall sequence identity to an NLR protein.

In embodiments, the nanobody is integrated internally within the NLR protein, and may partially or completely replace the integrated domain. The integrated domain of the wild-type NLR protein may be a HMA domain or a AvrRpt cleavage/NOI domain, preferably a HMA domain.

In embodiments, the NLR protein may be a Pik-1, RGA5 or Pii-2 NLR protein. Preferably the NLR protein is Pik-1. In embodiments, the Pik-1 protein may be rice (Oryza sativa) Pik-1; alternatively, the NLR protein may be from wheat (Triticum aestivum) or wild rice (Oryza brachyantha). In embodiments the NLR protein is from a crop plant, including grasses (eg, oats, barley), maize, banana, brassicas (eg cabbage, kale, broccoli), legumes (eg soybean, alfalfa, peas), Malvaceae (cotton, cacao), solanaceae (eg potatoes, tomatoes, pepper, aubergines), coffee. Pik-1 exists in rice in a number of alleles, including Pikh, Pikm, Pikp. A preferred rice allele is Pikm, although the inventors believe any of the alleles will be effective. Further, it will be appreciated that it is not necessary for the source of the NLR protein to be the same species as that where the pikobody is intended to be used - that is, a pikobody derived from rice Pik-1 can be used in plants other than rice.

In preferred embodiments the nanobody is a camelid nanobody.

In embodiments the chimeric protein includes a plant immune receptor (preferably an NLR protein) linked to two or more nanobodies, preferably wherein each nanobody is specific for a different pathogen effector protein.

A further aspect of the invention provides a chimeric protein having the domain structure CC-nanobody-NB-LRR. Preferably the CC-NB-LRR domains are derived from a plant NLR protein. Preferably the CB-NB-LRR domains are Pik-1 domains. In embodiments, the CC domain is at least 60%, 70%, 75%, 80%, 85%, 86%, 87%, 88%, 89%, 90%, 91%, 92%, 93%, 94%, 95%, 96%, 97%, 98%, 99%, or 100% identical to SEQ ID NO: 28. In embodiments, the LRR domain is at least 60%, 70%, 75%, 80%, 85%, 86%, 87%, 88%, 89%, 90%, 91%, 92%, 93%, 94%, 95%, 96%, 97%, 98%, 99%, or 100% identical to SEQ ID NO: 29. In embodiments, the NB domain is at least 60%, 70%, 75%, 80%, 85%, 86%, 87%, 88%, 89%, 90%, 91%, 92%, 93%, 94%, 95%, 96%, 97%, 98%, 99%, or 100% identical to SEQ ID NO: 30. In some embodiments, the order of the domains may be altered; for example, where the parent receptor protein has an integrated domain in a different region of the molecule, the nanobody (or other binding domain) may be placed there; for example, CC-NB-LRR-nanobody.

A further aspect of the invention provides a nucleic acid molecule comprising a nucleotide sequence encoding a chimeric protein as described herein. Also provided is a vector comprising a nucleic acid molecule as described herein. In a preferred embodiment, the nucleic acid sequences are operably linked to a regulatory sequence. Accordingly, in one embodiment, the nucleic acid sequences are expressed using a regulatory sequence that drives expression in specific cells or tissues. The regulatory sequences may comprise promoter sequences; in embodiments, the promoter sequences may be a tissue-specific promoter. Tissue specific promoters are transcriptional control elements that are active only in particular cells or tissues at specific times during plant development. In one example, the tissue-specific promoter is a fruit specific promoter. An example of a fruit-specific promoter is the E8 promoter. In another embodiment, the promoter is a root- or tuber-specific promoter.

Also provided is a plant cell comprising a chimeric protein, a nucleic acid molecule, or a vector, as described herein. Further provided is a plant or plant part comprising such a plant cell. "Plant part" includes reproductive material (seeds, spores, pollen), tissues, or organs, including flowers, fruit, roots, stems, leaves, tubers, bulbs, and so on.

Also provided is a method of producing a chimeric protein comprising linking a nanobody to a plant immune receptor, preferably an NLR protein.

The invention further provides a method of producing a modified plant cell, plant part or plant, wherein the method comprises introducing into a plant cell, or at least one plant cell of a plant part or plant, a chimeric protein, a nucleic acid molecule, or a vector, as described herein. The method may further comprise breeding the modified plant, or cell, or part, to produce offspring.

A further aspect of the invention provides a method of enhancing immunity of a plant against a pathogen, the method comprising providing a plant with a chimeric protein as described herein. The method may comprise directly administering the protein to the plant - for example, by injection into a cell - or may comprise introducing nucleic acids as described herein and allowing the nucleic acids to be expressed. Said introduction may be transient (as, for example, mRNA), or may be more permanent (as, for example, introduction and integration into the genome).

Some plant immune receptors - including Pik-1 - exert their effect in combination with additional molecules. In the case of Pik-1, this pairs with Pik-2 to cause cell-specific cell death. In embodiments of the invention, therefore, the chimeric protein as described herein is provided in combination with another protein which is acted upon by the chimeric protein. The other protein may be Pik-2. Thus, the nucleic acid sequences and vectors described herein may further comprise nucleic acid sequences coding for the other protein (eg, Pik-2). Such further sequences may be on the same or a different vector, and/or may be under the control of the same or a different promoter sequence. Other NLR pairs which may be useful in generating Pikobodies include Pias-1/Pias-2 or RGA4/RGA5 and their alleles/homologs. Note that the location of the integrated domain in these and other NLR pairs may differ from Pik-1. Where the target plant is one in which Pik-2 is expressed naturally, it may not be necessary to provide both Pik-1 and Pik-2 to obtain a desired effect; for example, targeted cell death. In some embodiments, gene editing may be used to alter a native Pik-1 protein in the plant (for example, by inserting the relevant nanobody sequence into the native gene); here, the native Pik-2 may be sufficient to ensure that the Pikobody is effective. It will be appreciated that a combination of Pik-1 and Pik-2 may be provided in any suitable manner; for example, by delivery of exogenous nucleic acid encoding the two proteins, or delivery of the proteins themselves.

Further provided is a plant protection product (eg, pesticide) comprising a chimeric protein, nucleic acid sequence, or vector, as described herein.

### Description of the Figures

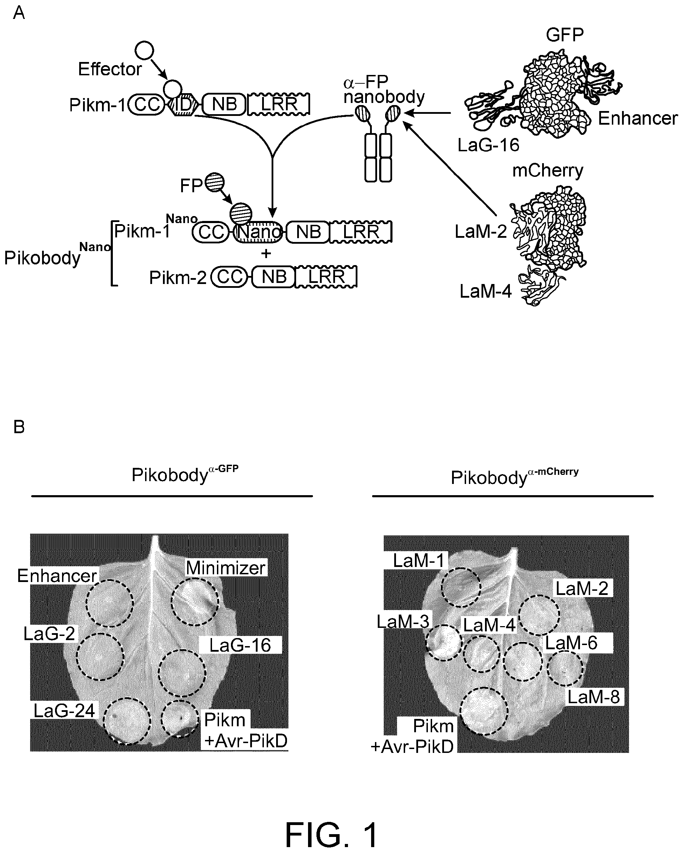

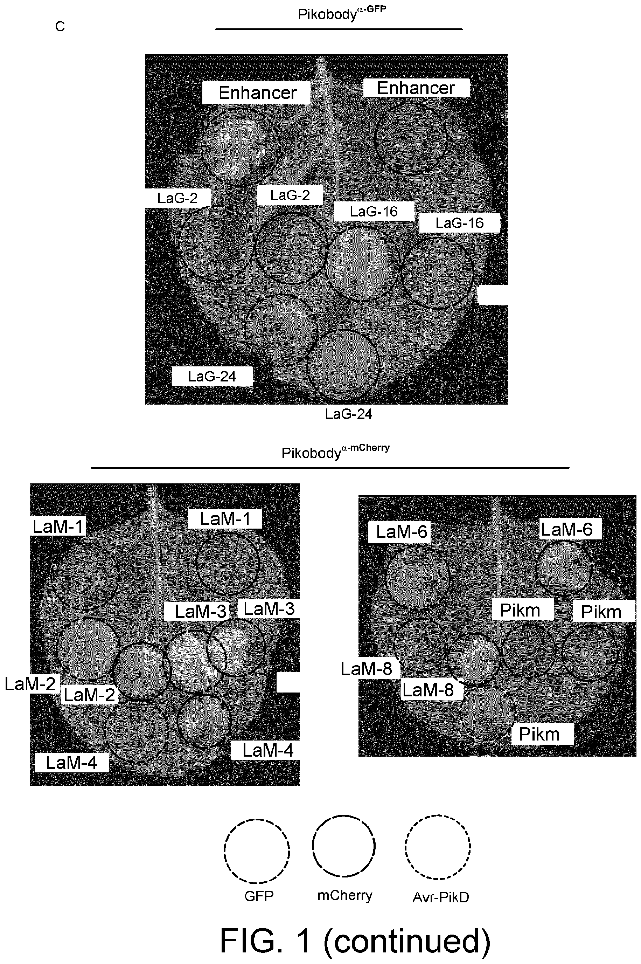

**Fig. 1****. Plant immune receptor-nanobody fusions trigger a hypersensitive cell death response in presence of the corresponding fluorescent protein antigen.** (**A**) Engineering of fluorescent protein (FP)-activated NLR sensor. The integrated HMA domain of the NLR Pikm-1, which is involved in pathogen effector recognition by direct binding, was swapped with nanobodies binding GFP of mCherry. Structures of the interaction interfaces of GFP (green) and the GFP-binding nanobodies Enhancer (3K1K) (Kirchhofer *et al.,* 2010) and LaG-16 (6LR7) (Zhang *et al.,* 2020) (light green) or mCherry (orange) and the mCherry-binding nanobodies LaM-2 (6IR2) and LaM-4 (6IR1) (purple). **(B)** Screen for autoimmunity of engineered Pikobody^{α-GFP/α-mCherry} upon overexpression in *N. benthamiana.* Representative *N. benthamiana* leaves infiltrated with appropriate constructs photographed 5 days after infiltration. The infiltration site for each construct is labelled on the picture and circled with a white dashed line. Autoimmunity is characterized by a hypersensitive cell death response (HR, cell-death) at the infiltration site (e.g. Minimizer), whereas no autoimmunity is observed if the leaf remains green at the infiltration site (e.g. Enhancer). Pikm-1/Pikm-2 pair (Pikm on the figure) co-infiltrated with AVR-PikD was used as a positive control for HR. (C) Pikobody^{α-GFP/α-mCherry} co-expressed with the corresponding fluorescent protein antigen results in a specific HR. Representative *N. benthamiana* leaves infiltrated with appropriate constructs photographed 5 days after infiltration. The infiltration site for each construct is labelled on the picture and circled with a yellow, orange or white dashed line where GFP, mCherry or AVR-PikD was co-infiltrated with the Pikobody, respectively. Pikm-1/Pikm-2 pair (Pikm on the figure) co-infiltrated with AVR-PikD was used as a positive control for HR.

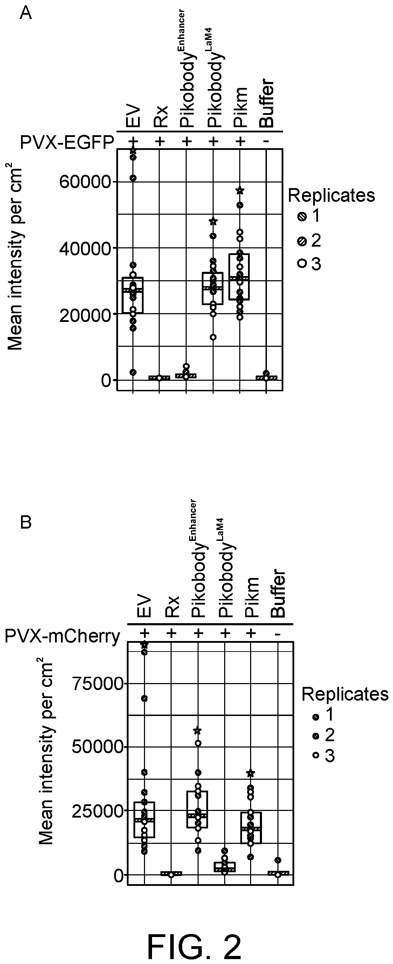

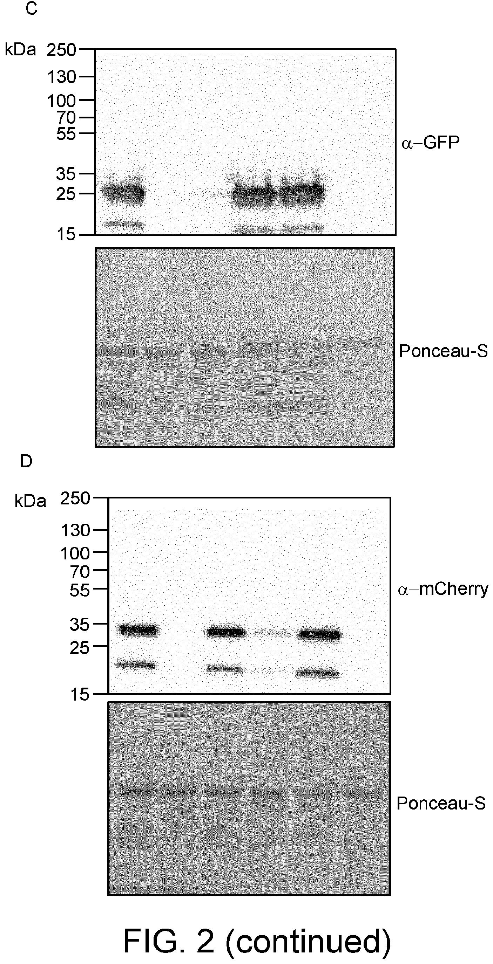

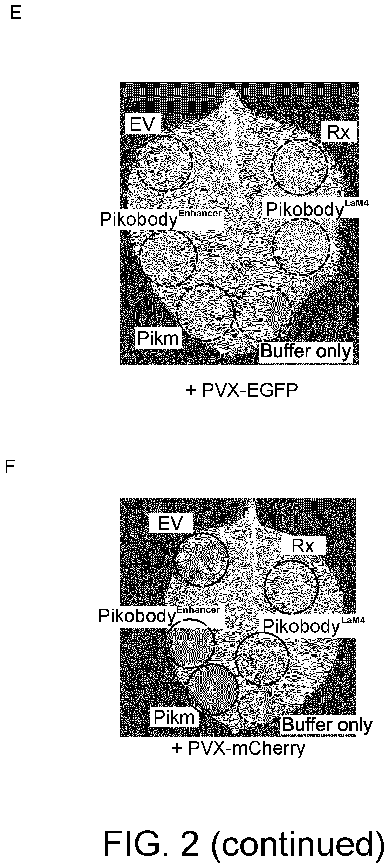

**Fig. 2****. Pikobodies confer resistance against engineered Potato Virus X strains expressing matching fluorescent proteins. (A-B)** Specific reduction in fluorescence intensity of PVX expressed EGFP (GFP) or mCherry in the presence of Pikobody^{Enhancer} or Pikobody^{LaM-4}, respectively. GFP **(A)** or mCherry **(B)** mean fluorescence intensity per cm² measured in *N. benthamiana* leaves 4 days post-infiltration and used as a proxy for PVX viral load in each infiltration site. The boxplot summarizes data obtained in three independent experiments (replicates) with six infiltration site per construct and per experiment. Red asterisks show significant differences between buffer only (no PVX added, negative control) and tested constructs in the presence of PVX-GFP **(A)** or PVX-mCherry **(B)** (Dunnett's test, pvalue < 0.001). Empty vector was used as a positive control for viral load and buffer only as a negative control for viral load. Rx, a resistance gene recognizing the coat protein from PVX, was used as a positive control for PVX resistance. **(C-D)** Specific reduction of PVX expressed GFP or mCherry accumulation in the presence of Pikobody^{Enhancer} or Pikobody^{LaM-4}, respectively. GFP or mCherry accumulation was used as proxy to evaluate viral load from PVX-GFP (C) or PVX-mCherry **(D).** For the immunoblot analysis, total protein was extracted 4 days after inoculation with PVX-GFP (C) or PVX-mCherry (D) in the presence of the tested constructs. Empty vector was used as a positive control for viral load and Buffer only as a negative control. GFP and mCherry were detected using anti-GFP or anti-mCherry antibody, respectively. Ponceau S staining shows equal protein loading across samples. **(E-F)** Specific HR triggered by PVX expressed GFP or mCherry in the presence of Pikobody^{Enhancer} or Pikobody^{LaM-4}, respectively. Representative *N. benthamiana* leaves infiltrated with appropriate constructs photographed 8 days after infiltration. The infiltration site for each construct is labelled on the picture and circled with a yellow, red or white dashed line for PVX-GFP (E), PVX-mCherry **(F)** or no PVX, respectively. The purple color on the infiltration sites in **(F)** is due to a high accumulation of mCherry.

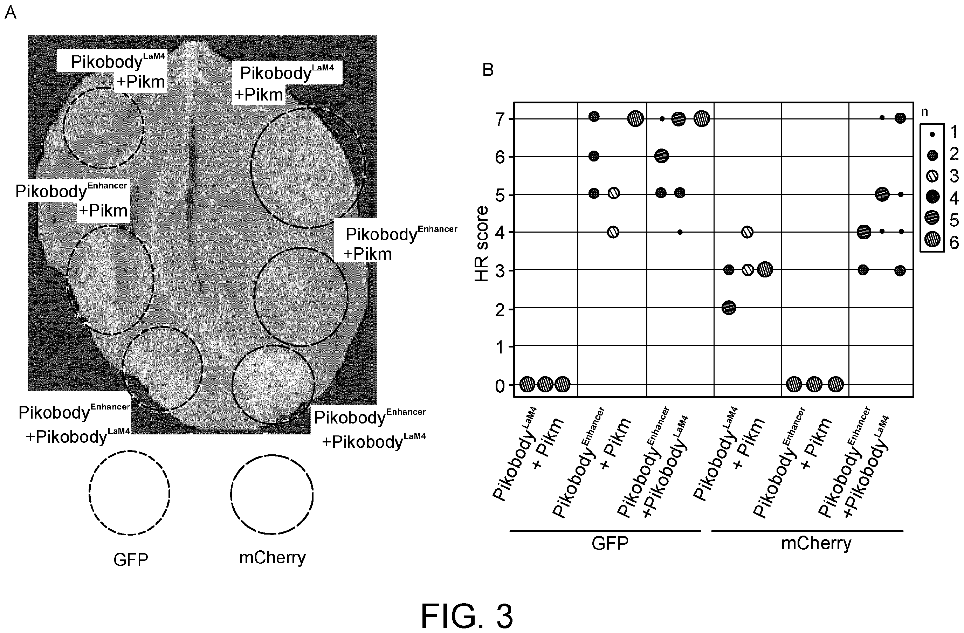

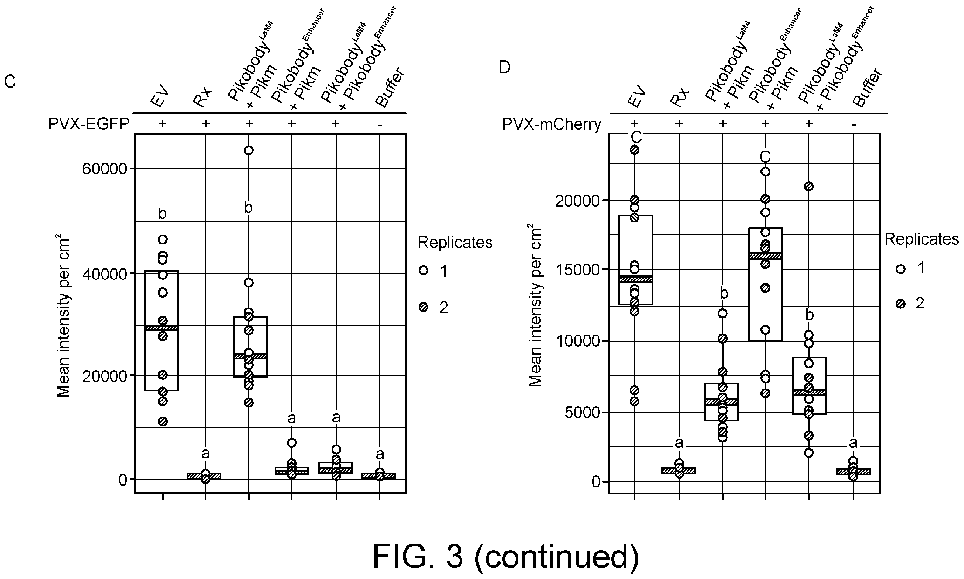

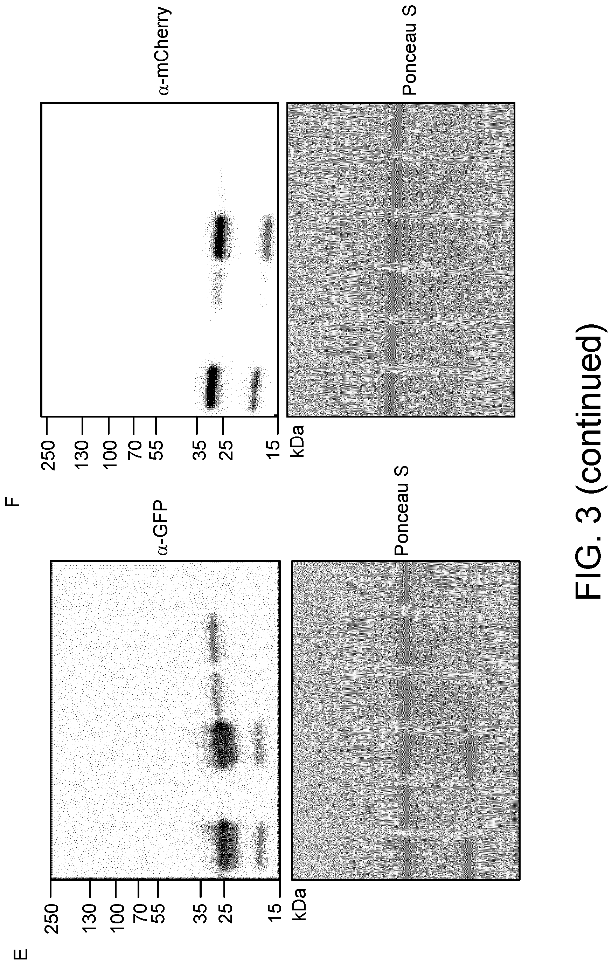

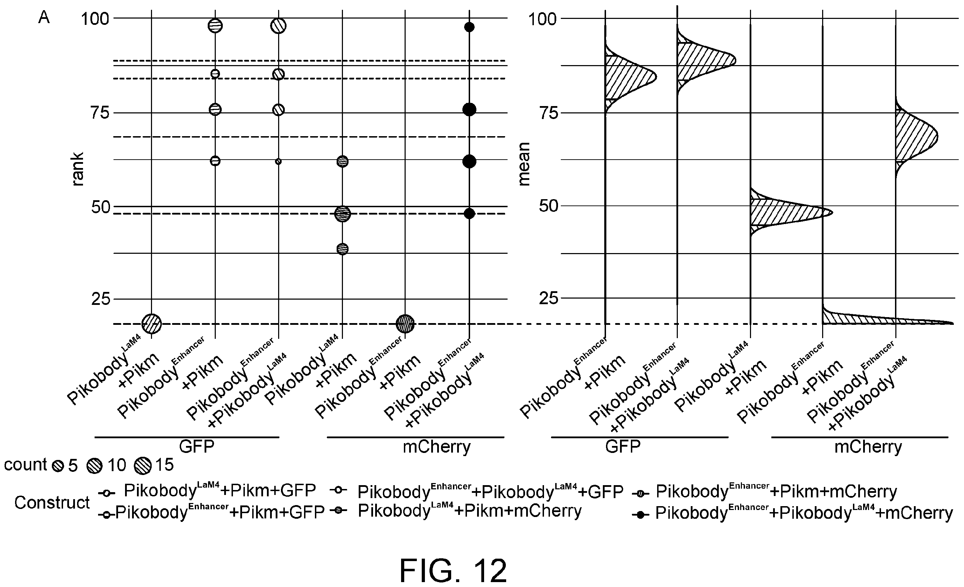

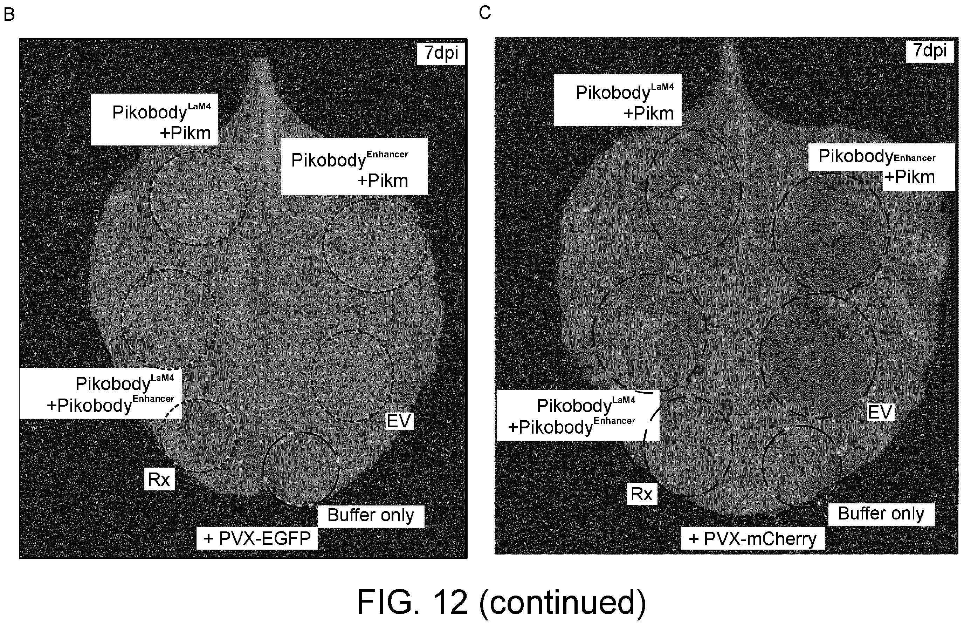

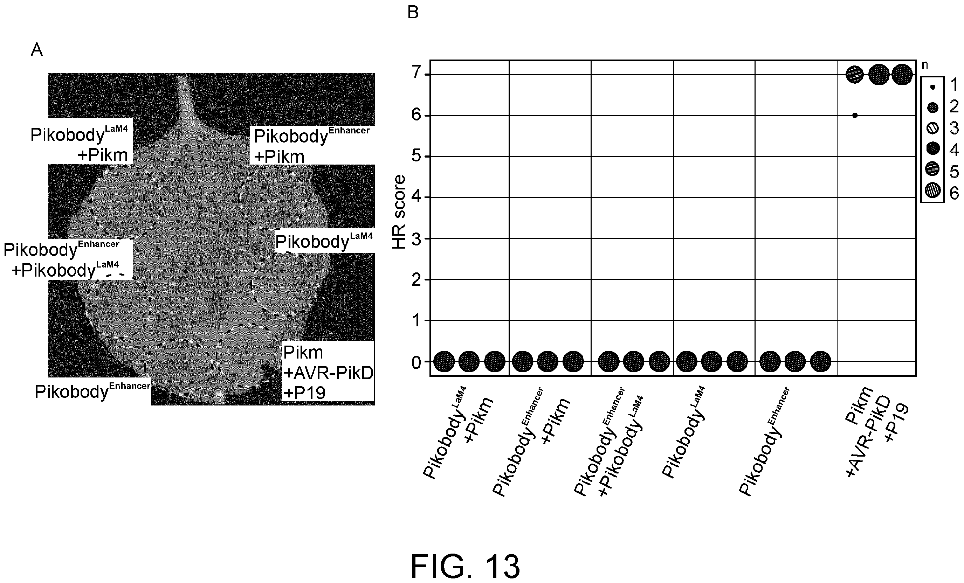

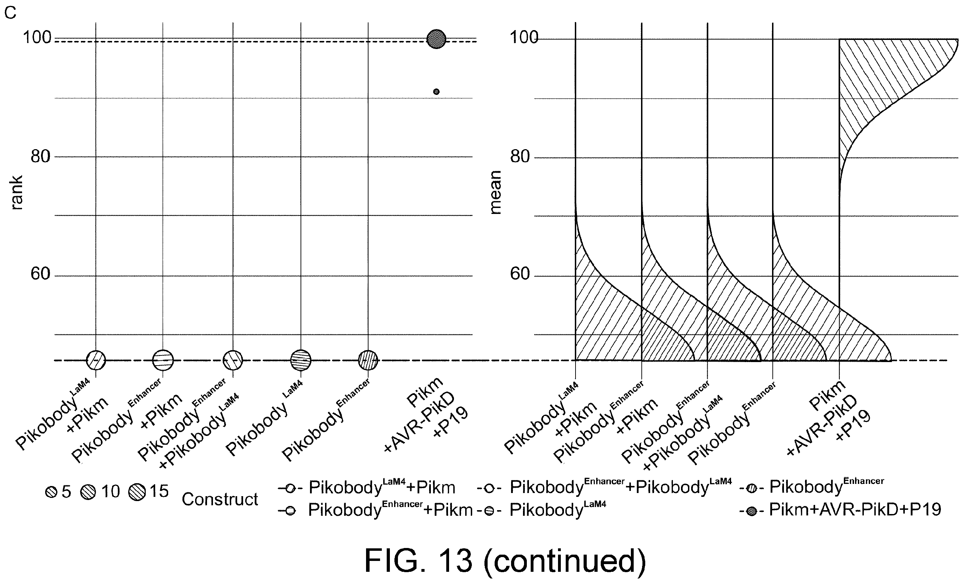

**Fig. 3****. Pikobodies are suitable for stacking. (A)** Pikobody stacking results in additive immune recognition. Representative *N. benthamiana* leaf infiltrated with constructs indicated in white and infiltration sites circled with a yellow or orange dashed line for GFP or mCherry, respectively. Leaves were photographed 4 days after infiltration. **(B)** HR scores visualized as dots plot, where the size of a dot is proportional to the number of samples with the same score (n) within the same replicate (1 to 3). The experiment was repeated three times with six internal replicates; each column represents a combination of constructs (labelled on the bottom). The statistical analyses of these results are presented in **Fig. 12** and the negative controls are shown in **Fig. 13****. (C-D)** Specific reduction in fluorescence intensity of PVX expressed GFP and mCherry in the presence of stacked Pikobody^{Enhancer} and Pikobody^{LaM-4}. GFP **(C)** or mCherry **(D)** mean fluorescence intensity per cm² measured in *N. benthamiana* leaves 4 days post-infiltration used as a proxy for PVX viral load in each infiltration site. The boxplot summarizes data obtained in two independent experiments (replicates) with six infiltration site per construct and per experiment. Empty vector was used as a positive control for viral load and buffer only as a negative control for viral load. Rx, a resistance gene recognizing the coat protein from PVX, was used as a positive control for PVX resistance. We performed two statistical tests on the presented data: a) Dunnett's test (pvalue < 0.001) showed significant differences between buffer only (no PVX added, negative control) and Empty vector, Pikobody^{LaM-4}+ Pikm in the presence of PVX-GFP (C) and showed significant differences between buffer only and all tested constructs but Rx in the presence of PVX-mCherry (C); b) ANOVA followed by Tukey HSD (pvalue < 0.05, letters depict the different groups) showed significant differences between buffer only (no PVX added, negative control) and Empty vector, Pikobody^{LaM-4}+ Pikm in the presence of PVX-GFP (C) and showed significant differences between buffer only and all tested constructs but Rx in the presence of PVX-mCherry and significant differences between Pikobody^{LaM-4}+ Pikm, Pikobody^{LaM-4}+ Pikobody^{Enhancer} and Empty vector, Pikobody^{Enhancer}+ Pikm (D). (E-F) Specific reduction of PVX expressed GFP and mCherry accumulation in the presence of stacked Pikobody^{Enhancer} and Pikobody^{LaM-4}. GFP or mCherry accumulation was used as proxy to evaluate viral load from PVX-GFP **(E)** or PVX-mCherry (F). For the immunoblot analysis, total protein was extracted 4 days after inoculation with PVX-GFP **(E)** or PVX-mCherry **(F)** in the presence of the tested constructs. Empty vector was used as a positive control for viral load and Buffer only as a negative control. GFP and mCherry were detected using anti-GFP or anti-mCherry antibody, respectively. Ponceau S staining shows equal protein loading across samples.

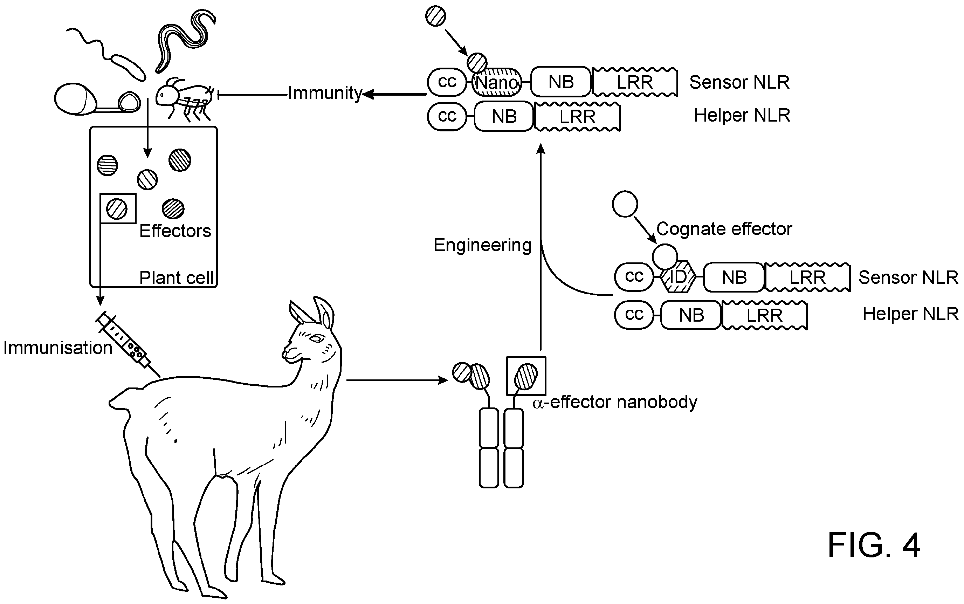

**Fig. 4****: Pipeline for the generation of functional Pikobodies.** Immunization of camelids with translocated pathogen effectors generates nanobodies which can be integrated into the Pikm scaffold in order to generate functional Pikobodies providing immunity against pathogens translocating these effectors.

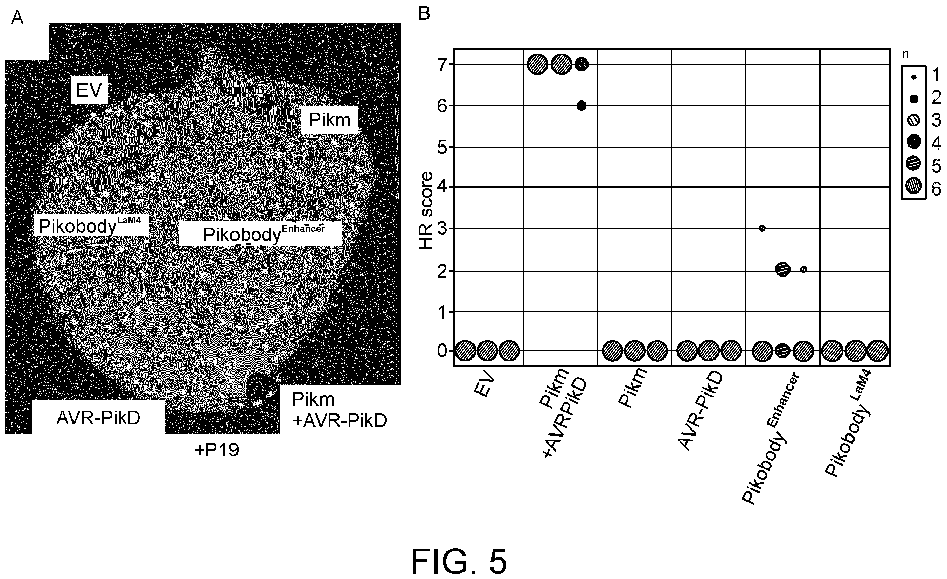

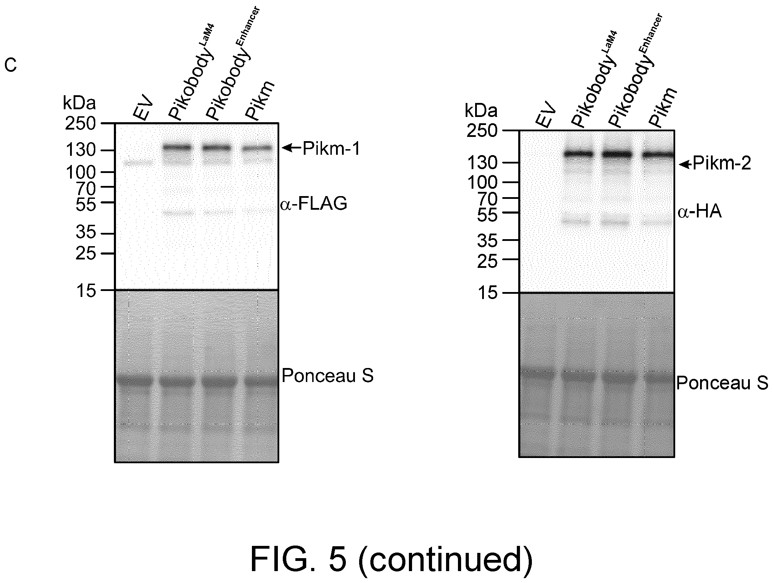

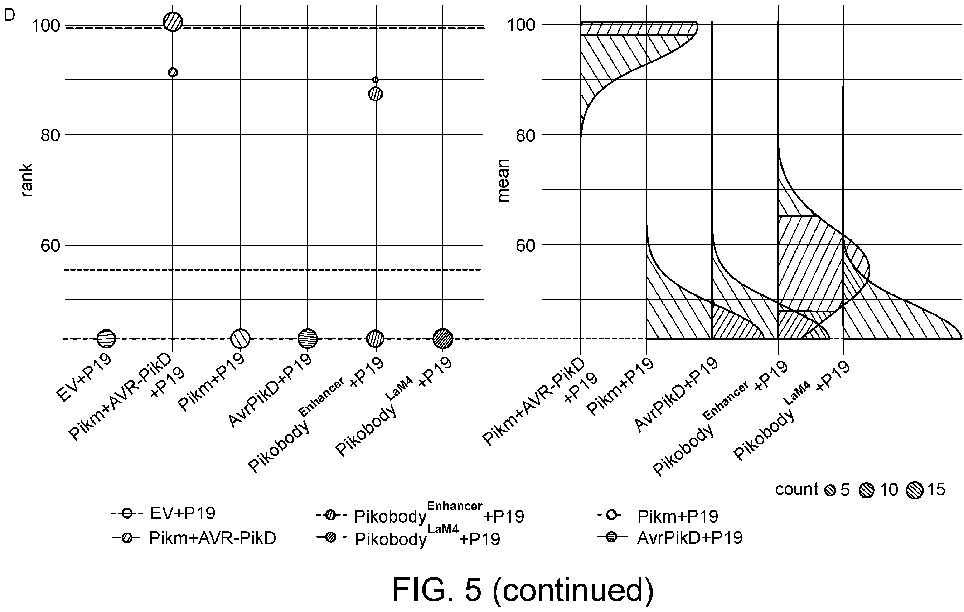

**Fig. 5****: Pikobody^{Enhancer} and Pikobody^{LaM4} are not autoactive. (A)** Representative *N*. *benthamiana* leaf infiltrated with constructs indicated in white and infiltration sites circled with a white dashed line, photographed 4 days after infiltration. **(B)** HR scores visualized as dots plot, where the size of a dot is proportional to the number of samples with the same score (n) within the same replicate (1 to 3). The experiment was repeated three times with six internal replicates; each column represents a combination of constructs (labelled on the bottom). The statistical analyses of these results are presented in (D). (C) Swapping the HMA domain for nanobodies does not affect protein accumulation. For the immunoblot analysis, total protein was extracted 2 days after transient expression of Pikobody^{Enhancer}, Pikobody^{LaM4} and the wild-type Pikm pair. Pikm-1 and the Pikm-1 Enhancer and LaM-4 were C-terminally 6xHis-3xFLAG-tagged and detected using anti-FLAG antibody, while Pikm-2 was C-terminally 6xHA-tagged and detected using anti-HA antibody. Empty vector was used as a negative control. The black arrow points to the band corresponding to Pikm-1 on the left and Pikm-2 on the right. **(D)** Statistical analysis of hypersensitive response showed in **(B)** conducted using an estimation method using besthr R library (MacLean, 2020). Empty vector combined with the silencing inhibitor p19 was used as a negative control, while the wild-type Pikm pair with the cognate effector AVR-PikD and the silencing inhibitor p19 was used as a positive control for HR. The left panels represent the ranked data (dots) and their corresponding mean (dashed line), with the size of a dot proportional to the number of observations with that specific value. The panels on the right show the distribution of 1000 bootstrap sample rank means, with the blue areas illustrating the 0.025 and 0.975 percentiles of the distribution. The difference is considered significant if the ranked mean for a given condition falls within or beyond the blue percentile of the mean distribution for another condition.

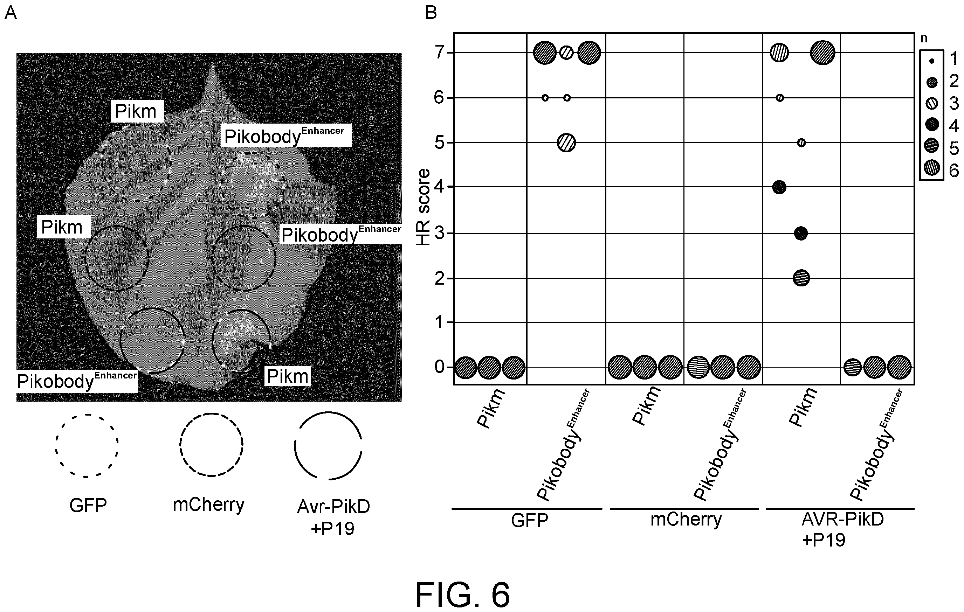

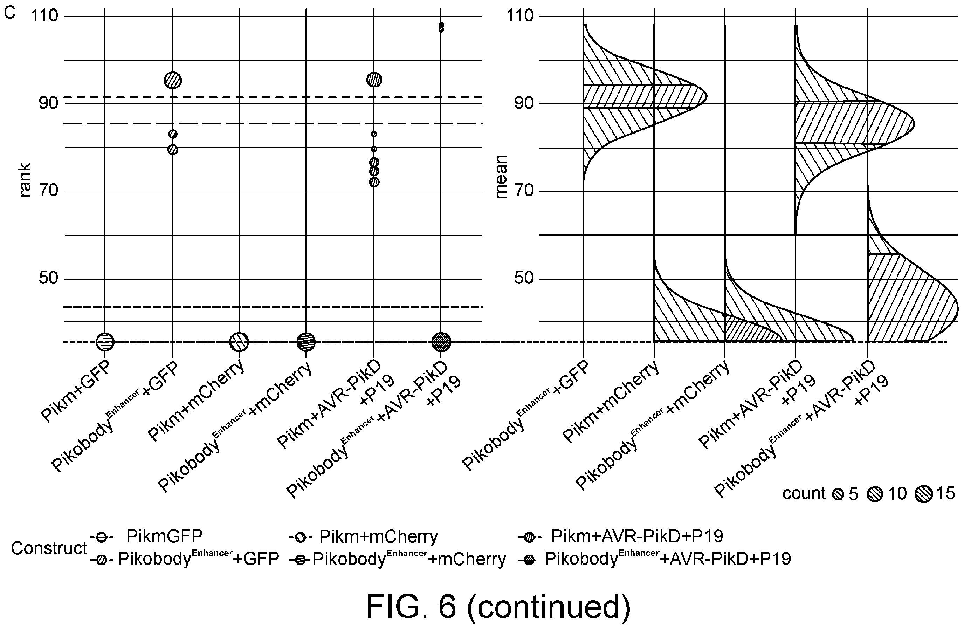

**Fig. 6****: Pikobody^{Enhancer} specifically triggers HR in response to GFP. (A)** Pikobody^{Enhancer} specifically recognizes GFP. Representative *N. benthamiana* leaf infiltrated with constructs indicated in white and infiltration sites circled with a dashed line, photographed 4 days after infiltration. Yellow, Orange or white color shows sites co-infiltrated with GFP, mCherry or AVR-PikD+P19, respectively. **(B)** HR scores visualized as dots plot, where the size of a dot is proportional to the number of samples with the same score (n) within the same replicate (1 to 3). The experiment was repeated three times with six internal replicates; each column represents a combination of constructs (labelled on the bottom). **(C)** Statistical analysis of hypersensitive response shown in **(B)** conducted using an estimation method using besthr R library (MacLean, 2020). The wild-type Pikm pair with either GFP or mCherry was used as a negative control, while the wild-type Pikm pair with the cognate effector AVR-PikD and the silencing inhibitor p19 was used as a positive control for HR. The left panels represent the ranked data (dots) and their corresponding mean (dashed line), with the size of a dot proportional to the number of observations with that specific value. The panels on the right show the distribution of 1000 bootstrap sample rank means, with the blue areas illustrating the 0.025 and 0.975 percentiles of the distribution. The difference is considered significant if the ranked mean for a given condition falls within or beyond the blue percentile of the mean distribution for another condition.

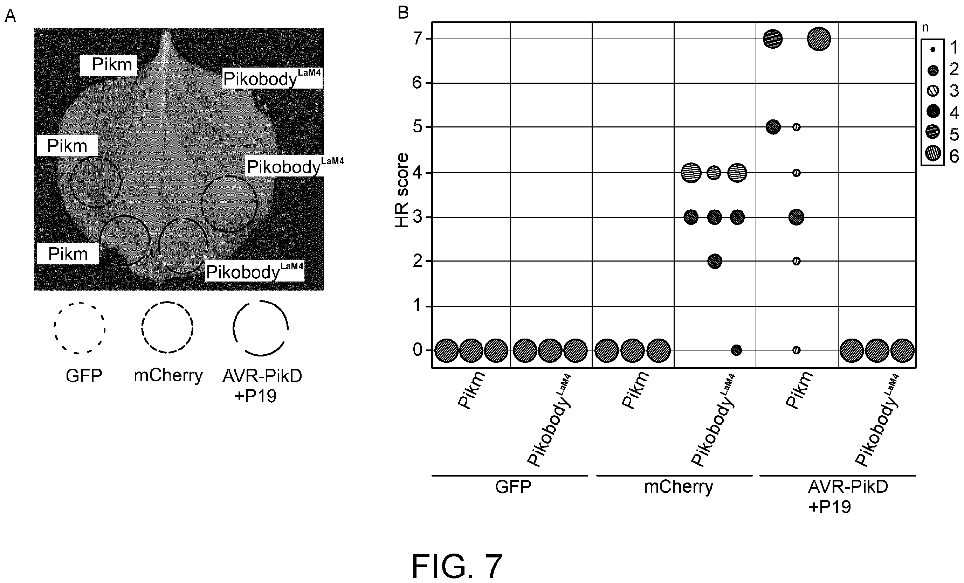

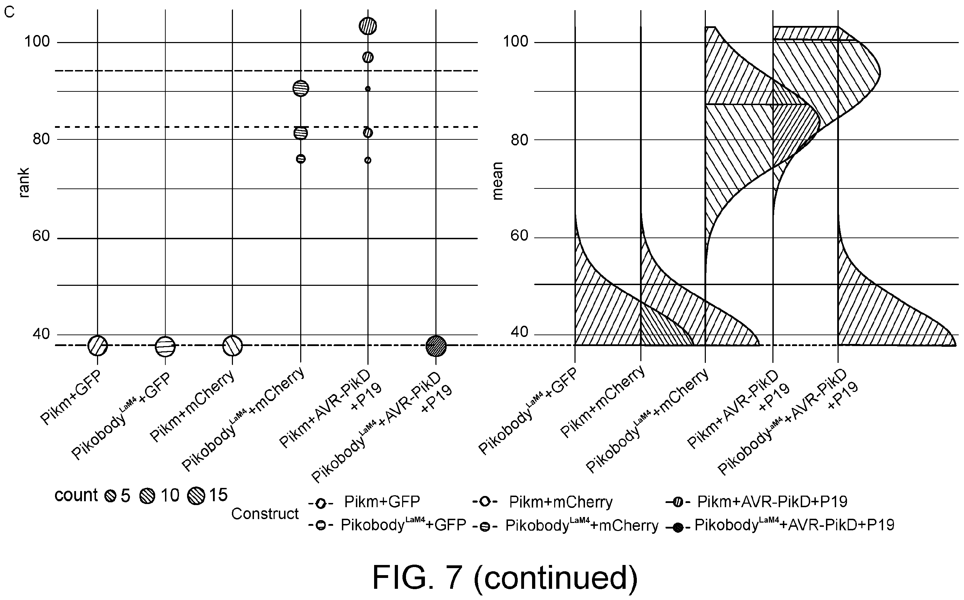

**Fig. 7****: Pikobody^{LaM4} specifically triggers HR in response to mCherry. (A)** Pikobody^{LaM-4} specifically recognizes mCherry. Representative *N. benthamiana* leaf infiltrated with constructs indicated in white and infiltration sites circled with a dashed line, photographed 4 days after infiltration. Yellow, Orange or white color show sites co-infiltrated with GFP, mCherry or AVR-PikD+P19, respectively. **(B)** and **(C)** are as for **Fig. 6****.**

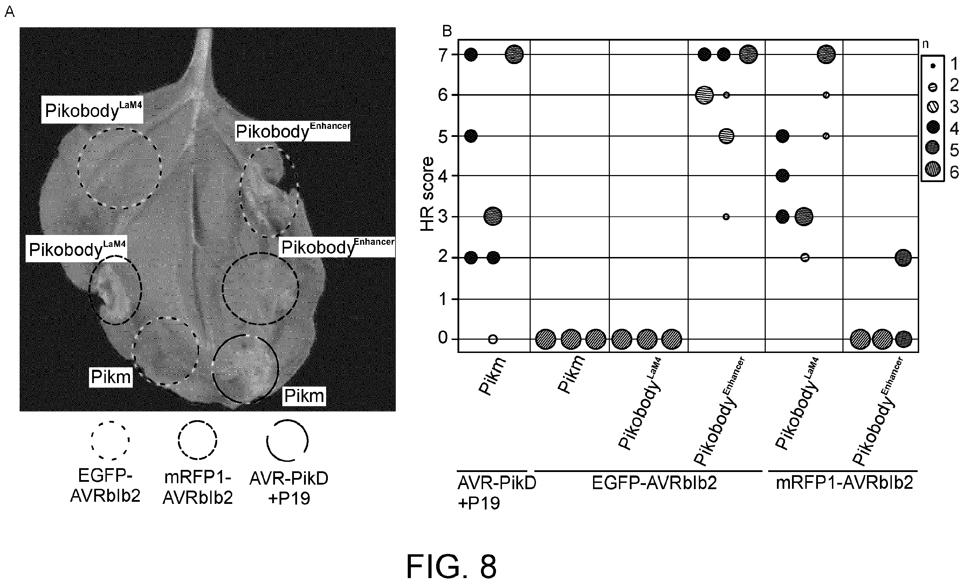

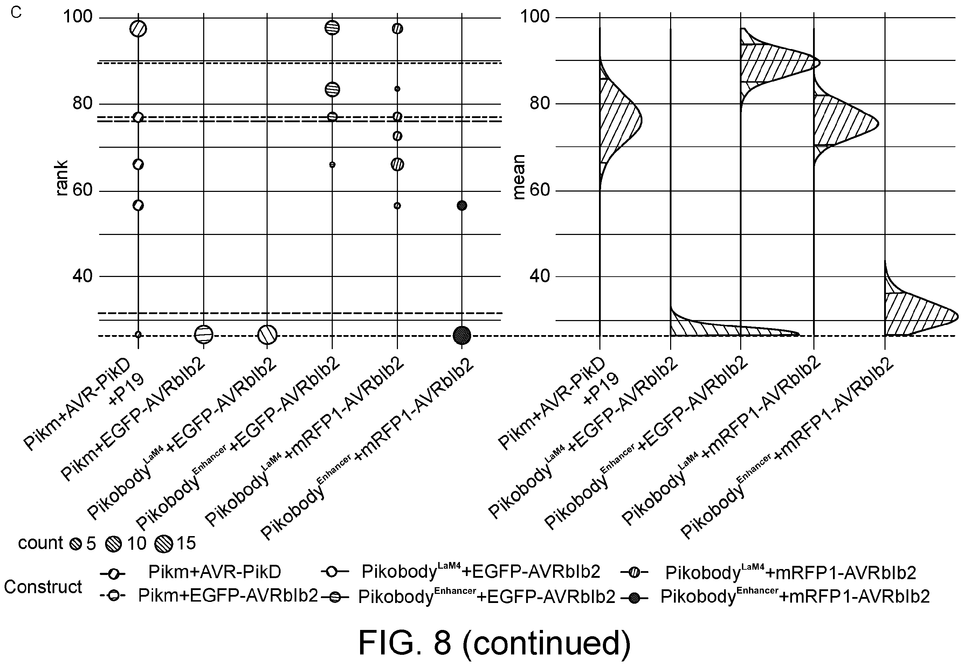

**Fig. 8****: Pikobodies specifically trigger HR in response to EGFP- or mRFP1-tagged *Phytophtora infestans* effector AVRblb2. (A)** Pikobody^{Enhancer} and Pikobody^{LaM4} specifically recognize EGFP- or mRFP1-tagged AVRblb2, respectively. Representative *N. benthamiana* leaf infiltrated with constructs indicated in white and infiltration sites circled with a dashed line, photographed 4 days after infiltration. Yellow, Orange or white color show sites co-infiltrated with EGFP-AVRblb2, mRFP1-AVRblb2 or AVR-PikD+P19, respectively. **(B)** and **(C)** are as for **Fig. 6****.**

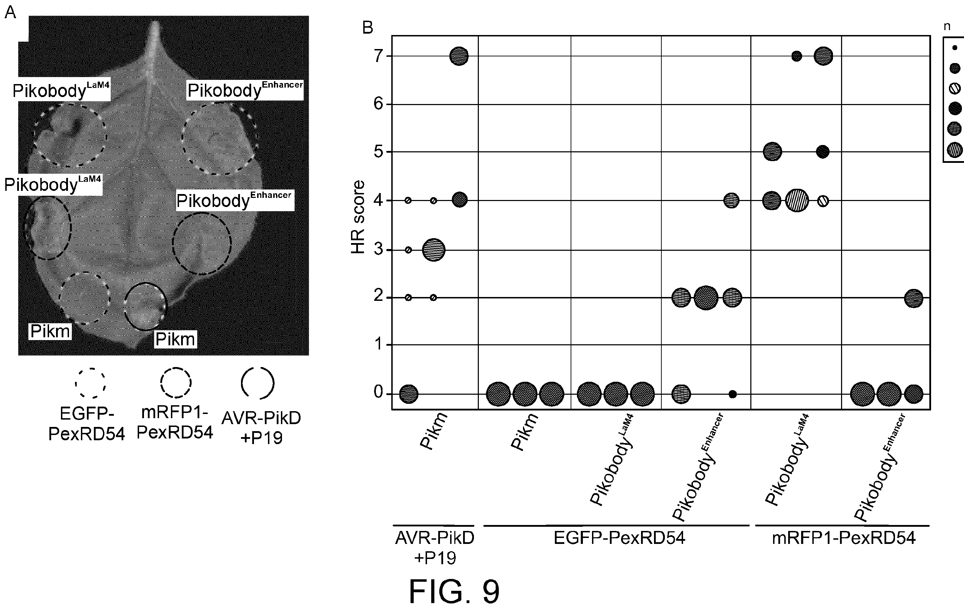

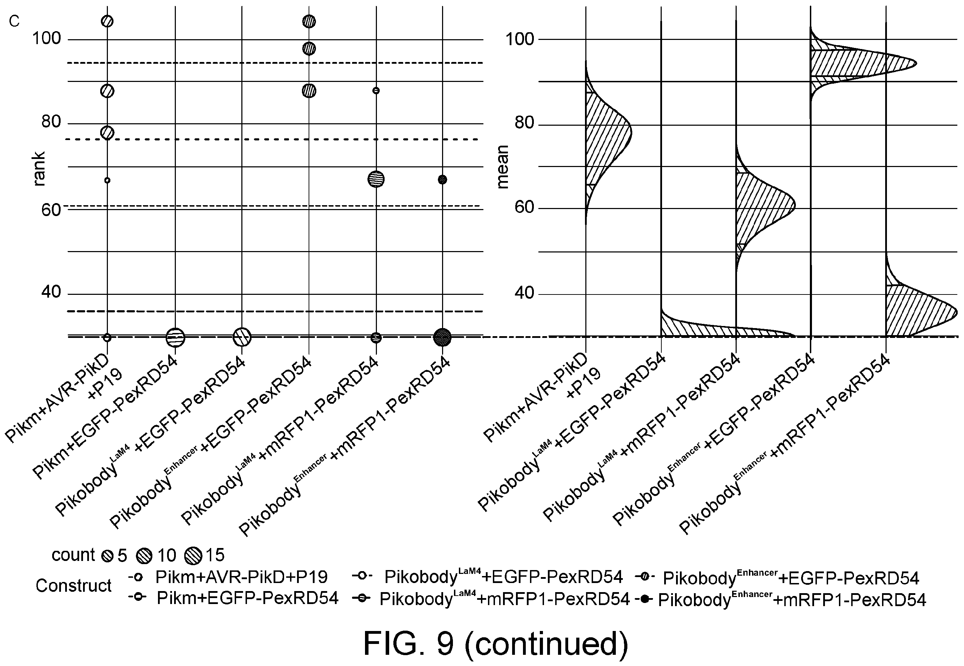

**Fig. 9****: Pikobodies specifically trigger HR in response to EGFP- and mRFP1-tagged *P. infestans* effector PexRD54. (A)** Pikobody^{Enhancer} and Pikobody^{LaM-4} specifically recognize EGFP- or mRFP1-tagged PexRD54, respectively. Representative N. *benthamiana* leaf infiltrated with constructs indicated in white and infiltration sites circled with a white dashed line, photographed 4 days after infiltration. Yellow, Orange or white color show sites co-infiltrated with EGFP-PexRD54, mRFP1-PexRD54 or AVR-PikD+P19, respectively. **(B)** and **(C)** are as for **Fig. 6****.**

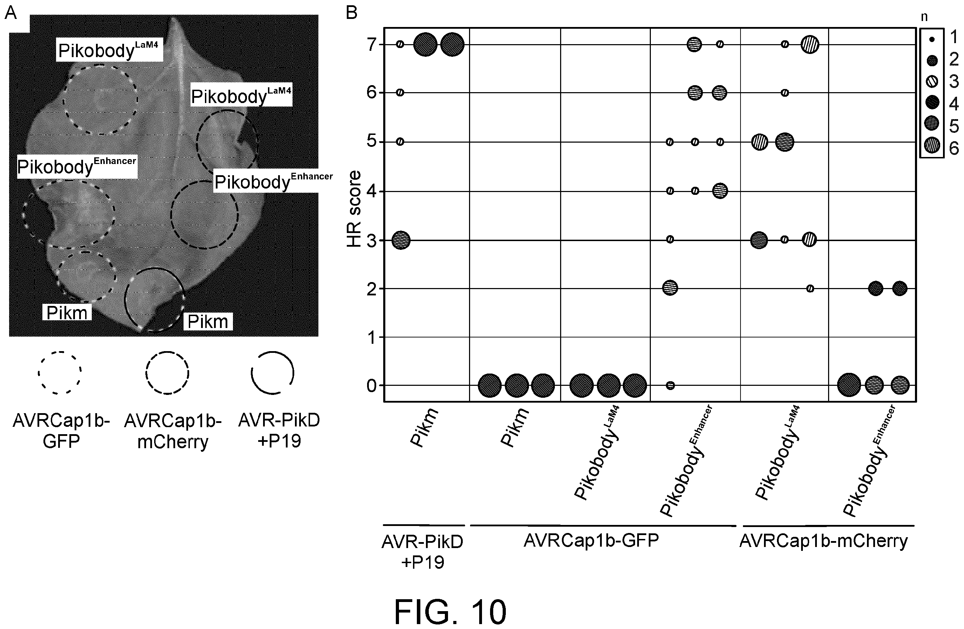

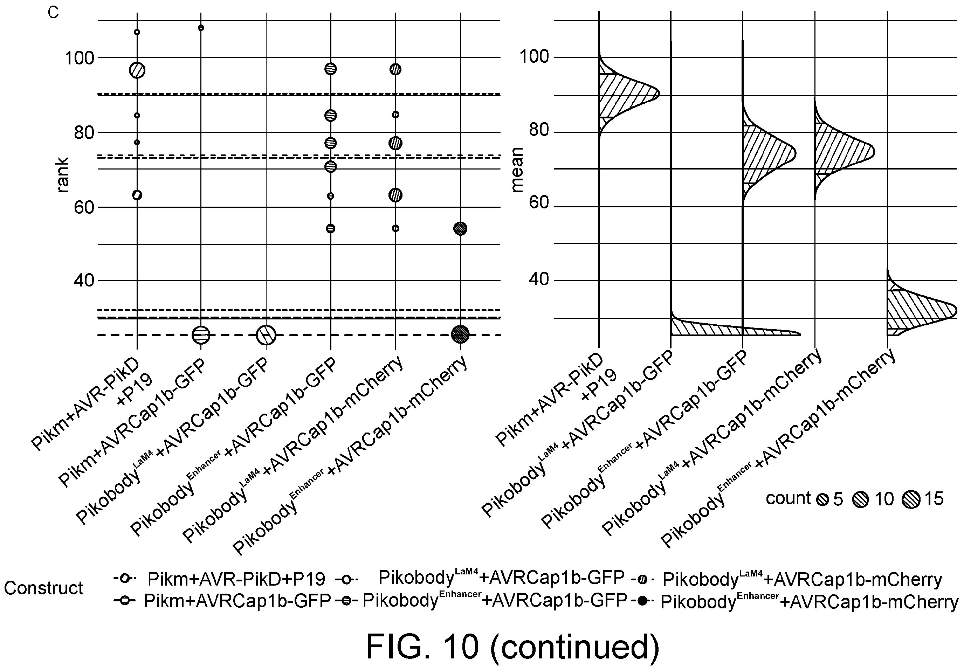

**Fig. 10****: Pikobodies specifically trigger HR in response to GFP- and mCherry-tagged *P. infestans* effector AVRCap1b. (A)** Pikobody^{Enhancer} and Pikobody^{LaM4} specifically recognize GFP- or mCherry-tagged AVRcap1b, respectively. Representative *N. benthamiana* leaf infiltrated with constructs indicated in white and infiltration sites circled with a white dashed line, photographed 4 days after infiltration. Yellow, Orange or white color show sites co-infiltrated with AVRCap1b-GFP, AVRCap1b-mCherry- or AVR-PikD+P19, respectively. **(B)** and **(C)** are as for **Fig. 6****.**

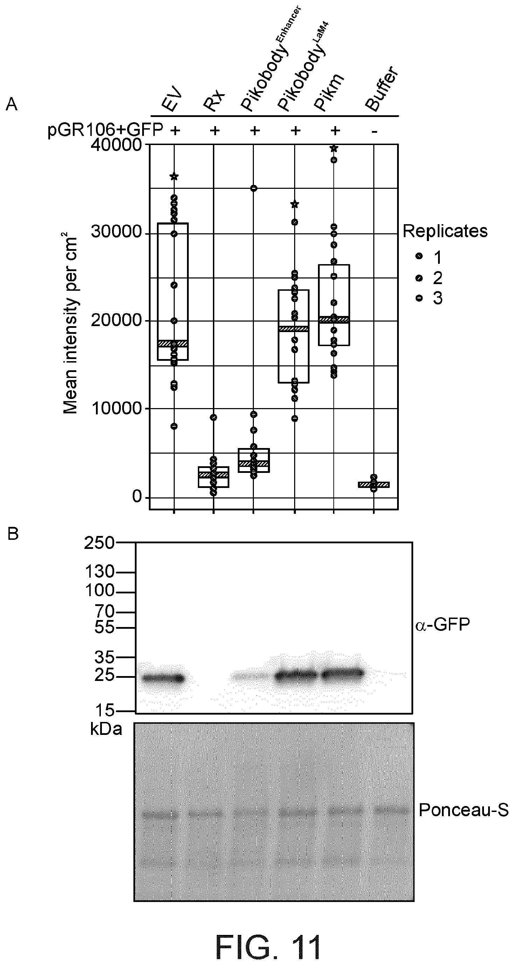

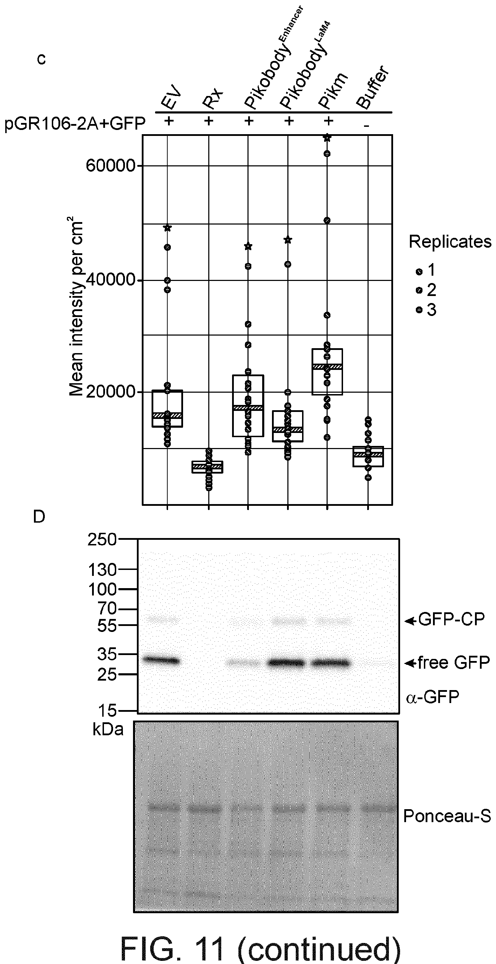

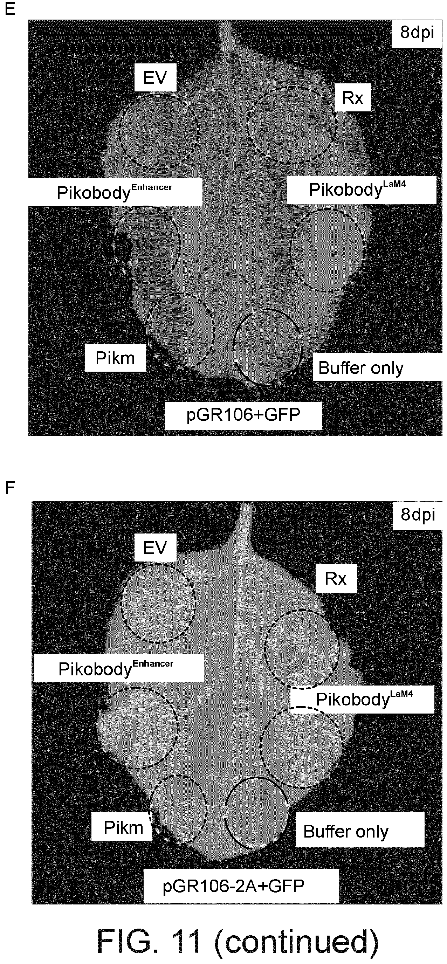

**Fig. 11****: Pikobody^{Enhancer} confers resistance against two other engineered variants of Potato Virus X expressing GFP. (A)** Specific reduction in fluorescence intensity of PVX expressed GFP in the presence of Pikobody^{Enhancer}. GFP mean fluorescence intensity per cm² measured in *N. benthamiana* leaves 4 days post-infiltration was used as a proxy for PVX viral load in each infiltration site for PVX engineered to express GFP from between the triple gene block and the coat protein (pGR106-GFP). The boxplot summarizes data obtained in three independent experiments (replicates) with six infiltration site per construct and per experiment. Red asterisks show significant differences between Buffer only (no PVX added, negative control) and tested constructs in the presence of pGR106-GFP (Dunnett's test, pvalue < 0.001). Empty vector was used as a positive control for viral load and Buffer only as a negative control for viral load. We also used Rx, a resistance gene recognizing the coat protein from PVX as a positive control for PVX resistance. **(B)** Specific reduction of PVX expressed GFP accumulation in the presence of Pikobody^{Enhancer}. GFP accumulation was used as proxy to evaluate viral load from pGR106-GFP. For the immunoblot analysis, total protein was extracted 2 days after inoculation with pGR106-GFP in the presence of the tested constructs. Empty vector was used as a positive control for viral load and Buffer only as a negative control. GFP was detected using anti-GFP antibody. Ponceau S staining shows equal protein loading across samples. **(C)** No reduction in fluorescence intensity of PVX expressed GFP fused to the coat protein in the presence of Pikobody^{Enhancer}. GFP mean fluorescence intensity per cm² measured in *N. benthamiana* leaves 4 days post-infiltration was used as a proxy for PVX viral load in each infiltration site for PVX engineered to express GFP fused to the coat protein using the porcine teschovirus-1 2A "self-cleaving" peptide (pGR106-2A-GFP) leading to approximately 50 % of free GFP and 50 % of GFP fused to the viral coat protein (CP). Boxplots and analysis as in **(A). (D)** Specific reduction of PVX expressed GFP accumulation fused to the coat protein in the presence of Pikobody^{Enhancer}. GFP accumulation was used as proxy to evaluate viral load from pGR106-2A-GFP. Immunoblot analysis as in **(B)** with black arrows pointing to GFP fused to coat protein (GFP-CP) and free GFP. **(E-F)** Specific HR triggered by the two version of PVX expressing GFP in the presence of Pikobody^{Enhancer}. Representative *N. benthamiana* leaves infiltrated with appropriate constructs photographed 8 days after infiltration. The infiltration site for each construct is labelled on the picture and circled with a yellow or white dashed line for pGR106+GFP (E) and pGR106-2A+GFP **(F)** or no PVX, respectively.
**Fig. 12****: Co-expressed Pikobodies are not autoactive. (A)** Stacking of Pikobodies does not result in autoimmunity. Representative *N. benthamiana* leaf infiltrated with constructs indicated in white and infiltration sites circled with a white dashed line, photographed 4 days after infiltration. **(B)** HR scores visualized as dots plot, where the size of a dot is proportional to the number of samples with the same score (n) within the same replicate (1 to 3). The experiment was repeated three times with six internal replicates; each column represents a combination of constructs (labelled on the bottom). Wild-type Pikm pair co-expressed with cognate effector AVR-PikD and silencer suppressor p19 was used as a positive control and Pikobody^{Enhancer} or Pikobody^{LaM4} expressed on their own as negative controls **(****Fig. 5****). (C)** Statistical analysis of hypersensitive response shown in **(B)** conducted using an estimation method using besthr R library (MacLean, 2020). Pikobody^{LaM4} was used as a negative control (Figure S1), while the wild-type Pikm pair with the cognate effector AVR-PikD and the silencing inhibitor p19 was used as a positive control for HR. The left panels represent the ranked data (dots) and their corresponding mean (dashed line), with the size of a dot proportional to the number of observations with that specific value. The panels on the right show the distribution of 1000 bootstrap sample rank means, with the blue areas illustrating the 0.025 and 0.975 percentiles of the distribution. The difference is considered significant if the ranked mean for a given condition falls within or beyond the blue percentile of the mean distribution for another condition.
**Fig. 13****: Statistical analysis of Pikobody stacking and HR phenotype. (A)** Statistical analysis of hypersensitive response shown in **Fig. 3** conducted using an estimation method using besthr R library (MacLean, 2020). The wild-type Pikm pair with Pikobody^{LaM4} and GFP was used as a negative control, while the wild-type Pikm pair with the cognate effector AVR-PikD and the silencing inhibitor p19 was used as a positive control for HR. **(B-C)** Specific HR triggered by PVX expressed EGFP (GFP, B) and mCherry (C) in the presence of stacked Pikobody^{Enhancer} and Pikobody^{LaM4}, respectively. Representative *N. benthamiana* leaves infiltrated with appropriate constructs photographed 7 days after infiltration. The infiltration site for each construct is labelled on the picture and all sites but "Buffer only" were co-infiltrated with PVX-GFP (left) or PVX-mCherry (right). Yellow, orange abd white colors depict the sites infiltrated with PVX-GFP, PVX-mCherry and no PVX, respectively. The purple color on the infiltration sites on (C) is due to a high expression of mCherry.

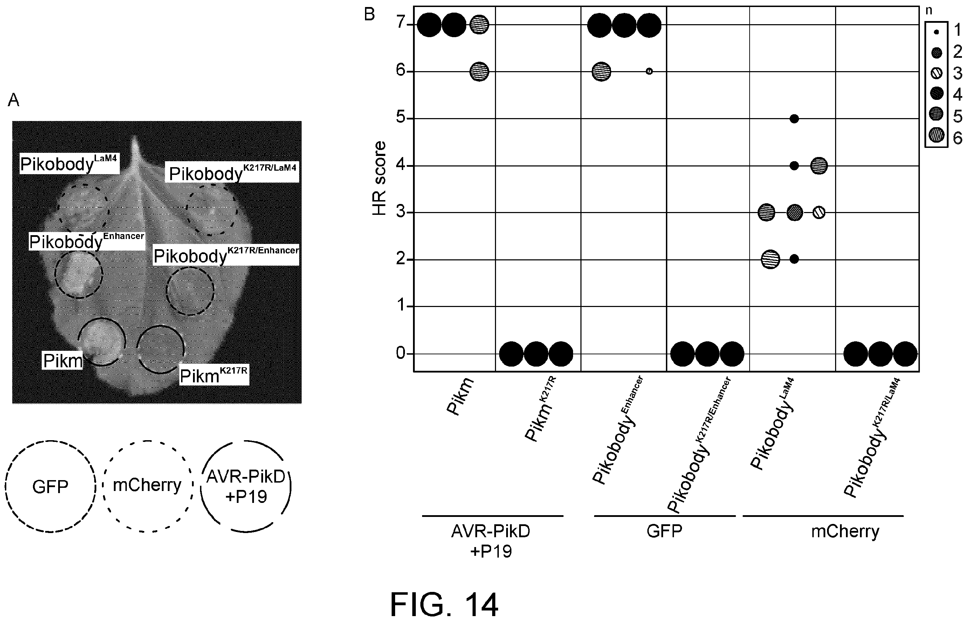

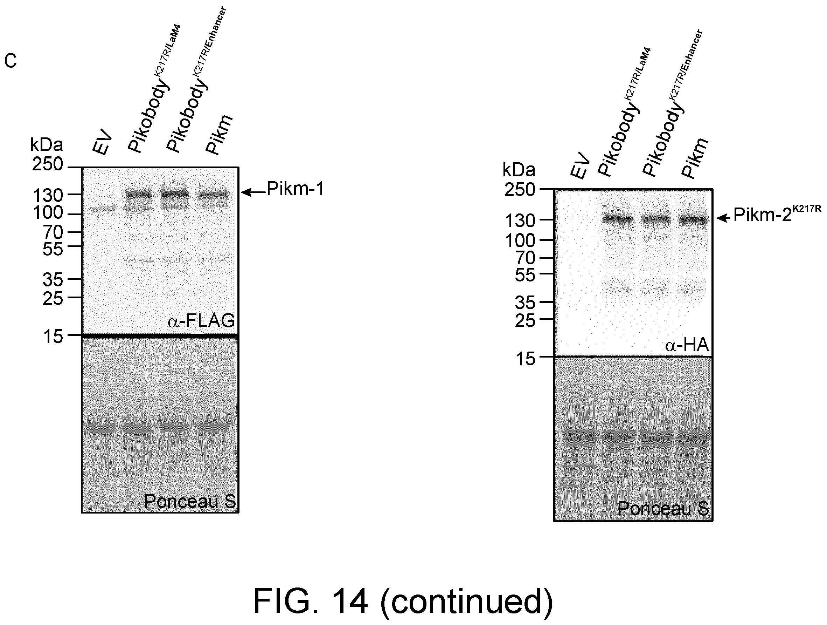

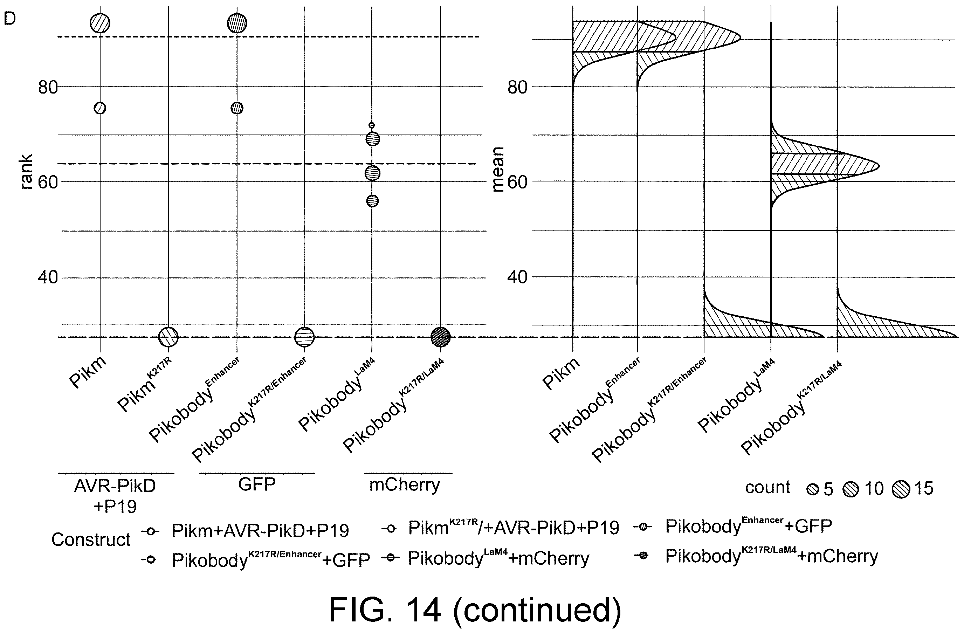

**Fig. 14****: Pikobody-mediated HR requires the ploop motif. (A)** An intact Pikm-2 P-loop motif is required for Pikobody-mediated HR. Representative *N. benthamiana* leaf infiltrated with constructs indicated in white and infiltration sites circled with a dashed line, photographed 4 days after infiltration. Yellow, Orange or white color shows sites co-infiltrated with GFP, mCherry or AVR-PikD+P19, respectively. Wild-type Pikm pair co-expressed with cognate effector AVR-PikD and silencer suppressor p19 was used as a positive control and Pikm^{K217R} P-loop mutant (mutation in Pikm-2) co-expressed with cognate effector AVR-PikD and silencer suppressor p19 was used as a negative control. **(B)** HR scores visualized as dots plot, where the size of a dot is proportional to the number of samples with the same score (n) within the same replicate (1 to 3). The experiment was repeated three times with six internal replicates; each column represents a combination of constructs (labelled on the bottom). The statistical analyses of these results are presented in **(D). (C)** Pikm-2^{K217R} P-loop mutant protein accumulates. For the immunoblot analysis, total protein was extracted 2 days after transient expression of Pikobody^{Enhancer}, Pikobody^{LaM4} and the wild-type Pikm pair. Pikm-1 and the Pikm-1 Enhancer and LaM-4 were C-terminally 6xHis-3xFLAG-tagged and detected using anti-FLAG antibody, while Pikm-2^{K217R} was C-terminally 6xHA-tagged and detected using anti-HA antibody. Empty vector was used as a negative control. Back arrows point to the band corresponding to Pikm-1 (anti-FLAG blot) and Pikm-2^{K217R} (anti-HA blot). **(D)** Statistical analysis of hypersensitive response showed in **(B)** conducted using an estimation method using besthr R library (MacLean, 2020). The Pikm pair with the Pikm-2^{K217R} P-loop mutant was used as as a negative control, while the wild-type Pikm pair with the cognate effector AVR-PikD and the silencing inhibitor p19 was used as a positive control for HR.

### Detailed Description

Engineered NLR-IDs often exhibit autoimmune activities in the absence of a ligand (Biatas *et al.,* 2021; De la Concepcion, Benjumea, *et al.,* 2021). This is possibly due to structural rearrangements induced when integrating a new domain or point mutations that perturb the resting state of the receptor. Hence, we first tested whether the Pik-1-nanobody fusions induce autoimmunity. Of the 11 tested fusions, six didn't exhibit autoimmunity when expressed with Pik-2 in leaves of the model plant *Nicotiana benthamiana* **(****Fig. 1B****)**, indicating that they can be used for follow-up gain-of-function assays. Next, we co-expressed these six fusions with GFP and mCherry. Among these, four produced a hypersensitive cell death response (HR, immune response readout) specifically when expressed with their matching fluorescent proteins (FPs) **(****Fig. 1C****,** Enhancer, LaG-16, LaM-4, and LaM-8). Additionally, a further three fusions which displayed weak autoimmunity gave a stronger hypersensitive cell death when combined with their matching fluorescent proteins **(****Fig. 1B****, C,** LaG-24, LaM-2, and LaM-6). This indicates that the Pik-1-VHH fusions are functional and can be endowed with new-to-nature activities. We termed this engineered immune receptor system Pikobody **(****Fig. 1****).**

We selected Pikobody^{Enhancer} (Pikm-2/Pikm-1^{Enhancer}) and Pikobody^{LaM4} (Pikm-2/Pikm-1^{LaM4}), recognising GFP and mCherry, respectively, to further confirm our results. We first challenged the absence of autoimmunity of these two Pikobodies by co-expressing them in *N. benthamiana* leaves with the gene silencing inhibitor p19, which is known to elevate heterologous expression levels (van der Hoorn *et al.,* 2003) **(****Fig. 5****).** Whereas Pikobody^{LaM4} did not produce any detectable HR, we could score a weak response in 5/18 samples for Pikobody^{Enhancer} when co-expressed with p19 **(****Fig. 5****).** These results confirm that these two Pikobodies are mainly inactive on their own and are appropriate for further experiments. Next, we co-expressed Pikobody^{Enhancer} and Pikobody^{LaM4} with GFP and mCherry and determined that they produced hypersensitive cell death only with their matching FP at levels similar to the response obtained with a natural combination of Pikm and a blast fungus effector **(****Fig. 6****,** **Fig. 7****).** We also noted that Pikobody^{Enhancer} and Pikobody^{LaM4} only respond to three pathogen effectors when they are tagged with the matching GFP/EGFP or mCherry/mRFP1 **(****Fig. 8****,** **Fig. 9****,** **Fig. 10****).** This further confirmed that the Pikobodies are functional FP sensors that detect FPs even when they are fused to another protein.

Can Pikobodies produce a functional immune response that is effective against a pathogen? We used recombinant *Potato virus X* (PVX) (Marillonnet *et al.,* 2008) expressing either GFP or mCherry to assay the ability of Pikobodies to reduce viral load **(Table 1).** These PVX variants express FPs from a duplicated coat protein sub-promoter in the virus genome. We used fluorescence intensity and immunodetection of GFP/mCherry accumulation as proxy for viral load in leaf samples **(****Fig. 2****).** Both Pikobody^{Enhancer} and Pikobody^{LaM4} specifically reduced fluorescent intensity of PVX expressed GFP or mCherry, respectively, to an extent comparable to that of Rx, an NLR known to confer immunity against PVX (Bendahmane *et al.,* 1999) **(****Fig. 2A****, B).** This reduction of fluorescence intensity correlates with reduced accumulation of virus expressed GFP or mCherry as compared to the empty vector control or wild-type Pikm-1/Pikm-2 **(****Fig. 2C****, D).** We did, however, observe a faint signal corresponding to GFP or mCherry in the samples with PVX-GFP or PVX-mCherry and Pikobody^{Enhancer} or Pikobody^{LaM4}, respectively, as compared to no detectable FP bands in the samples with Rx **(****Fig. 2C****, D).** In response to the matching PVX-FP, both Pikobodies triggered a clearly visible hypersensitive cell death further confirming that can produce a potent immune response to the recombinant viruses **(****Fig. 2E****, F).**

To independently confirm these results, we tested two additional recombinant PVX variants spliced in different ways to express GFP (Lu *et al.,* 2003; Cruz *et al.,* 1996) **(Table 1,** **Fig. 11****).** Pikobody^{Enhancer}, but not Pikobody^{LaM4}, markedly reduced GFP fluorescence intensity and protein accumulation when challenged with a PVX with the GFP sequence inserted between the triple gene block and coat protein in the virus genome **(****Fig. 11A****-B).** Pikobody^{Enhancer} didn't significantly affect fluorescence intensity when challenged with a PVX carrying a GFP in-frame fusion to the N-terminus of the virus coat protein and with an inserted porcine teschovirus-1 2A self-cleaving peptide **(****Fig. 11C**). However, Pikobody^{Enhancer}, but not Pikobody^{LaM4} and other control treatments, reduced the accumulation of virus expressed GFP **(****Fig. 11D**). This discrepancy could be explained by the observation that a GFP fraction remains fused to coat protein therefore somehow affecting fluorescence **(****Fig. 11D**) (Cruz *et al.,* 1996). Nonetheless, with both recombinant PVX-GFP, Pikobody^{Enhancer}, but not Pikobody^{LaM4}, caused a visible hypersensitive cell death **(****Fig. 11E****, F).** We conclude that Pikobody^{Enhancer} can provide enhanced resistance multiple recombinant PVX-GFP variants.

The simultaneous introduction of more than one plant immune receptor in a plant variety-a plant breeding strategy known as R gene stacking-can maximize resistance durability in the field by delaying the emergence of virulent pathogen races (Luo *et al.,* 2021). However, co-expression of plant immune receptors can lead to autoimmunity (Chae *et al.,* 2014; Tran *et al.,* 2017) or suppression of recognition (Hurni *et al.,* 2014). We investigated whether Pikobodies with different FP specificities are compatible with each other **(****Fig. 3****).** We first determined that none of three mis-matched combinations of Pikobodies induced autoimmunity **(****Fig. 12****)**. Co-expression of Pikobody^{Enhancer} or Pikobody^{LaM4} with the wild-type Pikm pair triggered hypersensitive cell death only in presence of GFP or mCherry, respectively, whereas co-expression of Pikobody^{Enhancer} and Pikobody^{LaM4} together produced a HR in the presence of both GFP and mCherry **(****Fig. 3A****-B,** **Fig. 13A****).** Similarly, co-expression of Pikobody^{Enhancer} and Pikobody^{LaM4} markedly reduced fluorescence intensity and protein levels of both GFP and mCherry expressed by recombinant PVX-FPs **(****Fig. 3C****-F).** The combination of Pikobody^{Enhancer} and Pikobody^{LaM4} also produced hypersensitive cell death in response to either PVX-GFP or PVX-mCherry **(****Fig. 13B****).** We conclude that Pikobody stacking can expand the response profile of these immune receptors.

We investigated the extent to which Pikobodies function through similar mechanisms as CC-NLRs and the wild-type Pik pair (Zdrzalek *et al.,* 2020). The conserved P-loop motif within the NB-ARC domain of CC-NLRs is required for the ADP/ATP switch that enables oligomerization into resistosome complexes (Wang, Wang, *et al.,* 2019)(Seshagiri and Miller, 1997; Chinnaiyan *et al.,* 1997; Li *et al.,* 1997; Dinesh-Kumar *et al.,* 2000). Pikobody^{K217R/Enhancer} and Pikobody^{K217R/LaM4} with a P-loop dead mutation in Pikm-2 (Pikm-2^{K217R}) failed to produce an HR to their corresponding FP even though the Pikm1 and Pikm2 proteins accumulated tosimilar levels as the wild-type immune receptors **(****Fig. 14****).** We conclude that the P-loop motif is required for Pikobody activity, and that the Pikobody system probably functions through the established mechanistic model of CC-NLRs.

### CONCLUSION

We built upon our growing understanding of the evolution and function of the Pik pair of NLRs (Bialas et al.; Zdrzalek et *al.,* 2020; de La Concepcion et al.) to use Pikm1 as a chassis for VHH nanobody fusions to engineer functional disease resistance genes with new-to-nature functionalities. This strategy to synthetic immune receptor engineering contrasts with earlier approaches, which were based on the modification of endogenous sequences and domains. Given that nanobodies can be readily generated to bind virtually any antigen, the Pikobody system has the potential to produce resistance genes against any pathogen or pest that delivers effectors inside host plant cells **(****Fig. 4****).**

### MATERIALS & METHODS

### Plant material and growth conditions

Wild type *N. benthamiana* were grown in Levingtons F2 compost in a glasshouse with set to 24 °C day / 22 °C night, humidity 45-65%, with supplementary lighting when the weather conditions required it.

### Plasmid constructions

The Golden Gate Modular Cloning (MoClo) kit (Weber *et al.,* 2011) and the MoClo plant parts kit (Engler *et al.,* 2014) were used for cloning, and all vectors are from this kit unless specified otherwise. Cloning design and sequence analysis were done using Geneious Prime (v2021.2.2; https://www.geneious.com). Plasmid construction is described in **Table 1.**

The Pikm-1 acceptor plasmid has been described in Bialas et *al.,* 2021. To generate Pikm-1:ancHMA fusions, ancHMA N2-I, ancHMAEMVKE, ancHMAFFE, ancHMASTSN, ancHMAVH, and ancHMAIVDPM were synthesised by GENEWIZ as Golden Gate modules. The ancHMAEMANK mutant was generated by amplification and fusion of the N-terminus of ancHMAEMVKE construct and the C-terminus of N2-I ancHMA variant. All ancHMA constructs corresponded to 187-264 residues of the full-length Pikm-1 protein and were subsequently assembled with custom-made p41308-PikmN (TSL SynBio) or p41308-PikmC (TSL SynBio) level 0 acceptors to generate Pikm-1:ancHMA fusions with or without a stop codon, respectively. Obtained modules were then used to generate Pikm-1:ancHMA expression constructs, featuring either N-terminal HA of C-terminal HF tags, by Golden Gate assembly using the same set of modules as previously used for Pikp-1 and pICH47732 binary vector. The plasmid was modified using the primers listed in Table 1. In the original plasmid residues 184-263 are swapped, while in the modified construct this is residues 188-258.

### Transient gene-expression and cell death assays

Transient gene expression in *N. benthamiana* were performed by agroinfiltration according to methods described by van der Hoorn *et al.* (2000). Briefly, *A. tumefaciens* strain GV3101 pMP90 carrying binary vectors were inoculated from glycerol stock in LB supplemented with appropriate antibiotics and grown O/N at 28 °C until saturation. Cells were harvested by centrifugation at 2000 x g, RT for 5 min. Cells were washed once and resuspended in infiltration buffer (10 mM MgCl₂, 10 mM MES-KOH pH 5.6, 200 µM acetosyringone) to the appropriate OD₆₀₀ (see **Table 1)** in the stated combinations and left to incubate in the dark for 2h at RT prior to infiltration into 5-week-old *N. benthamiana* leaves. Two leaves from three plants were inoculated per experiment (N = 6), and the experiment was repeated three times. Hypersensitive cell death phenotypes were scored 4-5 days post-infiltration in a range from 0 (no visible necrosis) to 7 (fully confluent necrosis) according to Adachi *et al.* (2019). The data was visualized with ggplot2 (v3.3.4; Ginestet, 2011) and the statistical analysis was performed using the R package besthr (v0.2.0; MacLean, 2020).

### Protein immunoblotting

Six *N. benthamiana* leaf discs (8 mm diameter) taken 2 days post agroinfiltration were homogenised in extraction buffer [10% glycerol, 25 mM Tris-HCI, pH 7.5, 1 mM EDTA, 150 mM NaCl, 2% (w/v) PVPP, 10 mM DTT, 1x protease inhibitor cocktail (SIGMA), 0.2% IGEPAL^{®} CA-630 (SIGMA)]. The supernatant obtained after centrifugation at 5,600 x g for 10 min at 4°C was used for SDS-PAGE. 4x SDS-PAGE sample buffer [final concentration: 50 mM Tris-HCI (pH 6.8), 100 mM DTT, 2% SDS, 0.01% bromophenol blue, 10% glycerol] was added and the samples were denatured by incubating at 72 °C for 10 min. Proteins were separated on 10-20%, SDS-PAGE gels (Bio-Rad) and transferred onto polyvinylidene difluoride (PVDF) membrane using a Trans-Blot turbo transfer system (Bio-Rad). Membranes were blocked in in 3% dried milk dissolved in Tris-buffered Saline [50mM Tris-HCL (pH7.5), 150mM NaCl] supplemented with 1% Tween^{®} 20 for 30 min before probing the membrane with either rat monoclonal anti-HA antibody (3F10, Roche) or mouse monoclonal ANTI-FLAG^{®} antibody conjugated to HRP (M2, Sigma) in a 1:4000 dilution. Chemiluminescent detection of signals after addition of either Pierce^{™} ECL Western (Thermo Fisher Scientific), or 1/5 SuperSignal^{™} West Femto Maximum Sensitivity Substrate (34095, Thermo Fisher Scientific) was done using the ImageQuant LAS 4000 luminescent imager (GE Healthcare Life Sciences). Equal loading was validated by staining the PVDF membranes with Ponceau S.

### PVX infection assays

Four to five-weeks old *N. benthamiana* plants were inoculated with engineered versions of *Potato virus X* (PVX) (Marillonnet *et al.,* 2008; Lu *et al.,* 2003; Cruz *et al.,* 1996) expressing either GFP or mCherry by agroinfiltration as described above. The PVX expression constructs are detailed in **Table 1.** Two leaves from three plants were inoculated per experiment (N = 6) as in **Fig. 2E****, F,** and the experiment was repeated three times. GFP or mCherry fluorescence was measured 4-5 days post-infiltration using the ImageQuant LAS 4000 luminescent imager (GE Healthcare Life Sciences) with the appropriate excitation and emission settings (EX: epi-RGB, EM:510DF10 filter for GFP and EX: epi-Green, EM: 605DF40 for mCherry). The mean fluorescence intensity per cm² in each infiltration site was measured using Fiji (ImageJ v2.1.0), and a Dunnett's test was conducted using the R package DescTools (v0.99.41; Signorell, 2021) to compare mean fluorescence intensity per cm² between buffer only (negative control) and all tested constructs followed by an ANOVA and Tukey HSD post-hoc test using the R package agricolae (v1.3-5). The data was visualized using ggplot2 (v3.3.4; Ginestet, 2011).

To determine GFP or mCherry accumulation upon PVX inoculation six leaf discs from the same infiltration site at the same time-point were taken and processed as above. Samples containing PVX expressed GFP or mCherry were diluted threefold in SDS-PAGE sample buffer to prevent saturation of the signal during detection. GFP and mCherry were probed with either mouse monoclonal anti-GFP antibody conjugated to HRP (B-2, Santa Cruz Biotech) or mouse monoclonal anti-mCherry TrueMAB^{™} antibody conjugated to HRP (OTI10G6, Thermo Fisher Scientific) at a 1:4000 or 1:2500 dilution, respectively. Chemiluminescent detection of signals was done as above.

### Tables

**Table 1: (overleaf)**Description of constructs and *Agrobacterium* strains.

Details of the constructs used in this study with associated selection antibiotic, purpose, publication, agrobacterium strain and OD₆₀₀ used for infiltrations.

| **Template** | **Relevant SEQ ID NO** | **Description** | **Selection** | **Name** | **Purpose** | **RE** | **Plasmids** | **Publication** | **Agro** | **OD** |
|---|---|---|---|---|---|---|---|---|---|---|
| - | - | pL0M-P5U-pMAS | Spec | pICH85281 | General LOM | - | - | Engler et *al.,* 2014 | - | - |
| - | - | pL0M-CT-6xHA | Spec | pICSL50009A | General LOM | - | - | TSL SynBio | - | - |
| - | - | pLOM-3UT-HSP18 | Spec | pICSL60008 | General LOM | - | - | TSL SynBio | - | - |
| - | - | pL0M-3UT-35Sterm | Spec | pICH41414 | General L0M | - | - | Engler et *al.,* 2014 | - | - |
| - | - | pL0M-P5U-2x35S-TMV | Spec | pICH51288 | General LOM | - | - | Engler et *al.,* 2014 | - | - |
| - | - | pL0V-CDS1 | Spec/LacZ | pICH41308 | Cloning | - | - | Weber et al., 2011 | - | - |
| - | - | pL0M-CT-6xHELLFIRE | Spec | pICSL50001 | General L0M | - | - | TSL SynBio | - | - |
| - | - | pL1V-F2 | Carb/LacZ | pICH47742 | Cloning | - | - | Weber et *al.,* 2011 | - | - |
| - | - | pL1V-F3 | Carb/LacZ | pICH47751 | Cloning | - | - | Weber et *al.,* 2011 | - | - |
| - | - | pL1V2-F1 | Kan/LacZ | pJK001c | Cloning | Bpil | pICSL4723, pICH47732, piCH41722 | Paulus et *al.,* 2020 | - | - |
| - | - | pL1V2-P19-F2 | Kan/LacZ | pJK268c | Cloning | Bpil | pISCL4723, pJK263, pICH47742, pICH41744 | Kourelis et *al.,* 2020 | - | - |
| - | - | pL2V | Kan/Crt | pICSL4723 | Cloning | - | - | Weber et *al.,* 2011 | - | - |
| - | - | pELE-3 | Spec | pICH41766 | ELE | - | - | Weber et *al.,* 2011 | - | - |
| - | - | pL1M-F1-Dummy | Carb | pICH54011 | Binary Agro L1M | - | - | Weber et *al.,* 2011 | - | - |
| - | - | pL0V-CDS1ns | Spec/LacZ | piCSL01005 | Cloning | - | - | Weber et al., 2011 | - | - |
| - | - | pL0V-CDS1ns-Pikm-1_acceptor | Spec/mRFP1 | Pikm-1_acceptor | Cloning | - | pICSL01005 | TSL SynBio | - | - |
| - | - | pL0V-CDS1ns-Pikm-1 | Spec | Pikm-1 | General LOM | Bpil | piCSL01005 | De la Concepcion et *al.,* 2018 | - | - |
| - | - | pL0V-CDS1ns-Pikm-2 | Spec | Pikm-2 | General LOM | Bpil | pICSL01005 | De la Concepcion et *al.,* 2018 | - | - |
| - | - | pGR106-PVX-GFP | Kan | pGR106-GFP | Binary Agro L2M | - | - | Wu et al., 2017 | GV3101 pMP90 | 0.001 |
| - | - | pGR106-PVX-F2A-GFP | Kan | pGR106-F2A-GFP | Binary Agro L2M | - | - | Cruz et *al.,* 1996 | GV3101 pMP90 | 0.005 and 0.05 |
| - | - | pL2M-2x35S::P19 | Kan | pJK050c | Binary Agro L2M | Bsal | pJK001c, pICH51288, pJK046, pICH41414 | Kourelis et *al.,* 2020 | GV3101 pMP90 | 0.25 |
| - | - | pICH31160-PVX | Kan | pICH31160 | Cloning | - | - | WO2008028661 (2008) | - | - |
| - | - | pK7WGF2-EGFP-PexRD54 | Spec | EGFP-PexRD54 | Binary Agro L2M | GW | pK7WGF2 | Dagdas et *al.,* 2016 | GV3101 pMP90 | 0.5 |
| - | - | pH7WGR2,0-mRFF1-PexRD54 | Spec | mRFP1-PexRD54 | Binary Agro L2M | GW | pH7WGR2,0 | Dagdas et *al.,* 2016 | GV3101 pMP90 | 0.5 |
| - | - | pK7WGF2-EGFP-AVRblb2 | Spec | EGFP-AVRblb2 | Binary Agro L2M | GW | pK7WGF2 | Bozkurt et *al.,* 2011 | GV3101 pMP90 | 0.5 |
| - | - | pH7WGR2,0-mRFF1-AVRblb2 | Spec | mRFP1-AVRblb2 | Binary Agro L2M | GW | pH7WGR2,0 | Bozkurt et *al.,* 2011 | GV3101 pMP90 | 0.5 |
| - | - | pICH47732-pMAS::GFP-AVRcaplb | Carb | GFP-AVRcaplb | Binary Agro L1M | Bsal | pICH47732, pICH85281, pICSL60008, pICSL30006, AVRcaplb | Derevnina et *al.,* 2021 | GV3101 pMP90 | 0.5 |
| - | - | pICH47732-pMAS::mCherry-AVRcaplb | Carb | mCherry-AVRcaplb | Binary Agro L1M | Bsal | pICH47732, pICH85281, pICSL60008, pICSL30003, AVRcaplb | Derevnina et *al.,* 2021 | GV3101 pMP90 | 0.5 |
| pL0V-CDSlns-Pikm-1_accepto r | 1, 2 | pL1V-F3-mRFP1 | Carb/mRFP1 | pICH47751_mR FP1 | Cloning | Bsal | pICH47751 | This work | - | - |
| pTL1 | 3, 4 | pL0M-CDS1-AvrPikD | Spec | pJK-0-023 | General LOM | Bsal/B pil | pICH41308 | This work | - | - |
| - | 5 | pL0M-CDS1ns-Enhancer_VHH | Spec | pJK-0-042 | General L0M | Bpil | pICSL01005 | This work | - | - |
| - | 6 | pL0M-CDS1ns-Minimizer_VHH | Spec | pJK-0-043 | General LOM | Bpil | pICSL01005 | This work | - | - |
| - | 7 | pL0M-CDS1ns-LaG-16_K/R_VHH | Spec | pJK-0-044 | General LOM | Bpil | piCSL01005 | This work | - | - |
| - | 8 | pL0M-CDS1ns-LaM-4_VHH | Spec | pJK-0-045 | General LOM | Bpil | piCSL01005 | This work | - | - |
| - | - | pL1M-F3-2×35S::Pikm-1-HF | Carb | pJK-B1-025 | Binary Agro L1M | Bsal | pICH51288, pICSLS0001, pICH41414, pICH47751, pL0M-CDSlns-Pikm-1 | This work | - | |
| - | - | pL1M-R2-pMAS::Pikm-2-6xHA | Carb | pJK-B1-036 | Binary Agro L1M | Bsal | pICH47811, pICH85281, pL0M-CDSlns-Pikm-2, pICSL50009 A, pICSL60008 | This work | - | - |
| - | - | pL2M-2x35S::EGFP | Kan | pJK-B2-022 | Binary Agro L2M | Bsal | pJK001c, pICH51288, pICH41414, pJK635 | This work | GV3101 pMP90 | 0.25 |
| - | - | pL2M-P19-2x35S::EGFP | Kan | pJK-B2-022p | Binary Agro L2M | Bsal | pJK268c, pICH51288, pICH41414, pJK635 | This work | GV3101 pMP90 | 0.25 |
| - | - | pL2M-2x35S::mCherry | Kan | pJK-B2-023 | Binary Agro L2M | Bsal | pJKOOlc, pICH51288, pICH41414, pJK636 | This work | GV3101 pMP90 | 0.5 |
| - | - | pL2M-P19-2x35S::mCherry | Kan | pJK-B2-023p | Binary Agro L2M | Bsal | pJK268c, pICH51288, pICH41414, pJK636 | This work | GV3101 pMP90 | 0.25 |
| - | - | pL2M-P19-2x35S::AvrPikD | Kan | pJK-B2-033p | Binary Agro L2M | Bsal | pJK268c, pICH51288, pICH41414, pJK-0-023 | This work | GV3101 pMP90 | 0.5 |
| - | - | pL2M-Pikm2-6xHA_2×35S::Pikm-1-HF | Kan | pJK-B2-099 | Binary Agro L2M | Bpil | pICSL4723, pICH54011, pICH41766, pJK-B1-025, pJK-B1-036 | This work | GV3101 pMP90 | 0.25 |
| - | - | pL2M-Pikm2_Pikm-1^LaG16 | Kan | pJK-B2-223M | Binary Agro L2M | Bsal | pJ K-C-045, pJK-0-044 | This work | GV3101 pMP90 | 0.25 |
| - | - | pL2M-Pikm2_Pikm-1^LaM4 | Kan | pJK-B2-224M | Binary Agro L2M | Bsal | pJK-C-045, pJK-0-045 | This work | GV3101 pMP90 | 0.25 |
| - | - | pL2M-Pikm2_Pikm-1^Enhancer | Kan | pJK-B2-280M | Binary Agro L2M | Bsal | pJK-C-045, pJK-0-042 | This work | GV3101 pMP90 | 0.25 |
| - | - | pL2M-Pikm2_Pikm-1^Minimizer | Kan | pJK-B2-301M | Binary Agro L2M | Bsal | pJK-C-045, pJK-0-043 | This work | GV3101 pMP90 | 0.25 |
| pJK-B2-224M | 9, 10 | pL2M-Pikm2_K217R_Pikm-1^LaM4 | Kan | pJK-B2-381 | Binary Agro L2M | Esp3I | - | This work | GV3101 pMP90 | 0.25 |
| pJK-B2-280M | 9, 10 | pL2M-Pikm2_K217R_Pikm-1^Enhancer | Kan | pJK-B2-383 | Binary Agro L2M | Esp3I | - | This work | GV3101 pMP90 | 0.25 |
| - | 11 | pL2M-Pikm2_Pikm-1^LaG2 | Kan | pJK-B2-480 | Binary Agro L2M | Bsal | pJ K-C-045 | This work | GV3101 pMP90 | 0.25 |
| - | 12 | pL2M-Pikm2_Pikm-1^LaG24 | Kan | pJK-B2-481 | Binary Agro L2M | Bsal | pJ K-C-045 | This work | GV3101 pMP90 | 0.25 |
| - | 13 | pL2M-Pikm2_Pikm-1^LaM1 | Kan | pJK-B2-482 | Binary Agro L2M | Bsal | pJK-C-045 | This work | GV3101 pMP90 | 0.25 |
| - | 14 | pL2M-Pikm2_Pikm-1^LaM2 | Kan | pJK-B2-483 | Binary Agro L2M | Bsal | pJK-C-045 | This work | GV3101 pMP90 | 0.25 |
| - | 15 | pL2M-Pikm2_Pikm-1^LaM3 | Kan | pJK-B2-484 | Binary Agro L2M | Bsal | pJK-C-045 | This work | GV3101 pMP90 | 0.25 |
| - | 16 | pL2M-Pikm2_Pikm-1^LaM6 | Kan | pJK-B2-485 | Binary Agro L2M | Bsal | pJK-C-045 | This work | GV3101 pMP90 | 0.25 |
| - | 17 | pL2M-Pikm2_Pikm-1^LaM8 | Kan | pJK-B2-486 | Binary Agro L2M | Bsal | pJK-C-045 | This work | GV3101 pMP90 | 0.25 |
| - | - | pL1V2-Pikm2-6xHA-F3 | Kan/mRFP1 | pJK-C-020 | Cloning | Bpil | pICSL4723, pICH54011, pICH41766, pICH47751_ mRFP1, pJK-B1-036 | This work | - | - |
| - | - | pL0V2-Pikm2_Pikm-1-HF_MCS | Kan/mRFP1 | pJ K-C-024 | Cloning | Bsal | pJK-C-020, pICH51288, pICH41414, pICSL50001, pLOM-CDSlns-Pikm-1_acceptor | This work | - | - |
| pICH4775 1_mRFP1 | 18, 19 | pL1V2-Pikm2_Pikm-1-HF_CDS1ns | Kan/mRFP1 | pJ K-C-045 | Cloning | Bpil | pJK-C-024 | This work | - | - |
| - | - | pICH31160-p35S::PVX-CP-EGFP | Kan | pJK-PVX-001 | Binary Agro PVX | Bsal | pICH31160, pJK635 | This work | GV3101 pMP90 | 0.001 |
| - | - | pICH31160-p35S::PVX-CP-mCherry | Kan | pJK-PVX-002 | Binary Agro PVX | Bsal | pICH31160, pJK636 | This work | GV3101 pMP90 | 0.001 |
| EC15095 | 20, 21 | pL0M-CDS1-EGFP | Spec | pJK635 | General LOM | Bpil | - | This work | - | - |
| EC15110 | 20, 21 | pL0M-CDS1-mCherry | Spec | pJK636 | General L0M | Bpil | - | This work | - | - |
| - | - | pELE-1 | Spec | pICH41722 | ELE | - | - | Weber et *al.,* 2011 | - | - |
| - | - | pL2M-Empty | Kan | pJK-B2-001 | Binary Agro L2M | Bpil | pICSL4723, pICH54011, pICH41722 | This work | GV3101 pMP90 | 0.25 |
| pJK-B2-099 | 22, 23 | pL2M-Pikm2_K217R-6xHA_2x35S::Pikm-1-HF | Kan | pJK-B2-386 | Binary Agro L2M | Esp3I | - | This work | GV3101 pMP90 | 0.25 |
| - | - | pL2M-2x35S::Rx-HF | Kan | pJK-B2-178-HF | Binary Agro L2M | Bsal | pJK001c, pICH51288, pICH41414, pICSL50001, pCR8-Rx | This work | GV3101 pMP90 | 0.25 |
| - | - | pCR8-CDS1ns-Rx | Spec | PCR8-Rx | General L0M | Bpil | piCSL01005 | Adachi et *al.,* 2019 | - | - |

### References

Adachi, H., Contreras, M.P., Harant, A., et al. (2019) An N-terminal motif in NLR immune receptors is functionally conserved across distantly related plant species. eLife, 8, e49956.
Ashikawa, I., Hayashi, N., Yamane, H., Kanamori, H., Wu, J., Matsumoto, T., Ono, K. and Yano, M. (2008) Two adjacent nucleotide-binding site-leucine-rich repeat class genes are required to confer Pikm-specific rice blast resistance. Genetics, 180, 2267-2276.
Bendahmane, A., Kanyuka, K. and Baulcombe, D.C. (1999) The Rx gene from potato controls separate virus resistance and cell death responses. Plant Cell, 11, 781-791.
Bialas, A., Langner, T., Harant, A., et al. (2021) Two NLR immune receptors acquired high-affinity binding to a fungal effector through convergent evolution of their integrated domain J. Monaghan, J. Kleine-Vehn, and P.-M. Delaux, eds. eLife, 10, e66961.
Cesari, S., Bernoux, M., Moncuquet, P., Kroj, T. and Dodds, P.N. (2014) A novel conserved mechanism for plant NLR protein pairs: the "integrated decoy" hypothesis. Plant-Microbe Interact., 5, 606.
Cesari, S., Thilliez, G., Ribot, C., et al. (2013) The rice resistance protein pair RGA4/RGA5 recognizes the Magnaporthe oryzae effectors AVR-Pia and AVR1-CO39 by direct binding. Plant Cell, 25, 1463-1481.
Cesari, S., Xi, Y., Declerck, N., et al. (2021) Design of a new effector recognition specificity in a plant NLR immune receptor by molecular engineering of its integrated decoy domain. bioRxiv, 2021.04.24.441256.
Chae, E., Bomblies, K., Kim, S.-T., et al. (2014) Species-wide genetic incompatibility analysis identifies immune genes as hot spots of deleterious epistasis. Cell, 159, 1341-1351.
Chinnaiyan, A.M., Chaudhary, D., O'Rourke, K., Koonin, E.V. and Dixit, V.M. (1997) Role of CED-4 in the activation of CED-3. Nature, 388, 728-729.
Cruz, S.S., Chapman, S., Roberts, A.G., Roberts, I.M., Prior, D.A. and Oparka, K.J. (1996) Assembly and movement of a plant virus carrying a green fluorescent protein overcoat. Proc. Natl. Acad. Sci., 93, 6286-6290.
Dangl, J.L., Horvath, D.M. and Staskawicz, B.J. (2013) Pivoting the plant immune system from dissection to deployment. Science, 341, 746-751.
De la Concepcion, J.C., Benjumea, J.V., Bialas, A., Terauchi, R., Kamoun, S. and Banfield, M.J. (2021) Functional diversification gave rise to allelic specialization in a rice NLR immune receptor pair. , 2021.06.25.449940.
De la Concepcion, J.C., Franceschetti, M., MacLean, D., Terauchi, R., Kamoun, S. and Banfield, M.J. (2019) Protein engineering expands the effector recognition profile of a rice NLR immune receptor T. Nürnberger, D. Weigel, and T. Nürnberger, eds. eLife, 8, e47713.
De la Concepcion, J.C., Franceschetti, M., Maqbool, A., Saitoh, H., Terauchi, R., Kamoun, S. and Banfield, M.J. (2018) Polymorphic residues in rice NLRs expand binding and response to effectors of the blast pathogen. Nat. Plants, 4, 576.
De la Concepcion, J.C., Maidment, J.H.R., Longya, A., Xiao, G., Franceschetti, M. and Banfield, M.J. (2021) The allelic rice immune receptor Pikh confers extended resistance to strains of the blast fungus through a single polymorphism in the effector binding interface. PLOS Pathog., 17, e1009368.
Dinesh-Kumar, S.P., Tham, W.-H. and Baker, B.J. (2000) Structure-function analysis of the tobacco mosaic virus resistance gene N. Proc. Natl. Acad. Sci., 97, 14789-14794.
Eitas, T.K. and Dangl, J.L. (2010) NB-LRR proteins: pairs, pieces, perception, partners, and pathways. Curr. Opin. Plant Biol., 13, 472-477.
Engler, C., Youles, M., Gruetzner, R., Ehnert, T.-M., Werner, S., Jones, J.D.G., Patron, N.J. and Marillonnet, S. (2014) A Golden Gate modular cloning toolbox for plants. ACS Synth. Biol., 3, 839-843.
Fridy, P.C., Li, Y., Keegan, S., et al. (2014) A robust pipeline for rapid production of versatile nanobody repertoires. Nat. Methods, 11, 1253-1260.
Giannakopoulou, A., Steele, J.F.C., Segretin, M.E., Bozkurt, T.O., Zhou, J., Robatzek, S., Banfield, M.J., Pais, M. and Kamoun, S. (2015) Tomato I2 immune receptor can be engineered to confer partial resistance to the oomycete Phytophthora infestans in addition to the fungus Fusarium oxysporum. Mol. Plant. Microbe Interact., 28, 1316-1329.
Ginestet, C. (2011) ggplot2: elegant graphics for data analysis. J. R. Stat. Soc. Ser. A Stat. Soc., 174, 245-246.
Greenberg, A.S., Avila, D., Hughes, M., Hughes, A., McKinney, E.C. and Flajnik, M.F. (1995) A new antigen receptor gene family that undergoes rearrangement and extensive somatic diversification in sharks. Nature, 374, 168-173.
Guo, L., Cesari, S., Guillen, K. de, et al. (2018) Specific recognition of two MAX effectors by integrated HMA domains in plant immune receptors involves distinct binding surfaces. Proc. Natl. Acad. Sci., 115, 11637-11642.
Hamers-Casterman, C., Atarhouch, T., Muyldermans, S., Robinson, G., Hammers, C., Songa, E.B., Bendahman, N. and Hammers, R. (1993) Naturally occurring antibodies devoid of light chains. Nature, 363, 446-448.
Hoorn, R.A.L. van der, Laurent, F., Roth, R. and Wit, P.J.G.M. de (2000) Agroinfiltration is a versatile tool that facilitates comparative analyses of Avr9/Cf-9-induced and Avr4/Cf-4-induced necrosis. Mol. Plant. Microbe Interact., 13, 439-446.
Hoorn, R.A.L. van der, Rivas, S., Wulff, B.B.H., Jones, J.D.G. and Joosten, M.H.A.J. (2003) Rapid migration in gel filtration of the Cf-4 and Cf-9 resistance proteins is an intrinsic property of Cf proteins and not because of their association with high-molecular-weight proteins. Plant J., 35, 305-315.
Hurni, S., Brunner, S., Stirnweis, D., Herren, G., Peditto, D., McIntosh, R.A. and Keller, B. (2014) The powdery mildew resistance gene Pm8 derived from rye is suppressed by its wheat ortholog Pm3. Plant J., 79, 904-913.
Kanzaki, H., Yoshida, K., Saitoh, H., Fujisaki, K., Hirabuchi, A., Alaux, L., Fournier, E., Tharreau, D. and Terauchi, R. (2012) Arms race co-evolution of Magnaporthe oryzae AVR-Pik and rice Pik genes driven by their physical interactions. Plant J., 72, 894-907.
Kim, S.H., Qi, D., Ashfield, T., Helm, M. and Innes, R.W. (2016) Using decoys to expand the recognition specificity of a plant disease resistance protein. Science, 351, 684-687.
Kirchhofer, A., Helma, J., Schmidthals, K., et al. (2010) Modulation of protein properties in living cells using nanobodies. Nat. Struct. Mol. Biol., 17, 133-138.
Könning, D., Zielonka, S., Grzeschik, J., et al. (2017) Camelid and shark single domain antibodies: structural features and therapeutic potential. Curr. Opin. Struct. Biol., 45, 10-16.
Kroj, T., Chanclud, E., Michel-Romiti, C., Grand, X. and Morel, J.-B. (2016) Integration of decoy domains derived from protein targets of pathogen effectors into plant immune receptors is widespread. New Phytol., 210, 618-626.
Le Roux, C., Huet, G., Jauneau, A., et al. (2015) A receptor pair with an integrated decoy converts pathogen disabling of transcription factors to immunity. Cell, 161, 1074-1088.
Li, P., Nijhawan, D., Budihardjo, I., Srinivasula, S.M., Ahmad, M., Alnemri, E.S. and Wang, X. (1997) Cytochrome c and dATP-dependent formation of Apaf-1/Caspase-9 complex initiates an apoptotic protease cascade. Cell, 91, 479-489.
Lu, R., Malcuit, I., Moffett, P., et al. (2003) High throughput virus-induced gene silencing implicates heat shock protein 90 in plant disease resistance. EMBO J., 22, 5690-5699.
Luo, M., Xie, L., Chakraborty, S., et al. (2021) A five-transgene cassette confers broadspectrum resistance to a fungal rust pathogen in wheat. Nat. Biotechnol., 39, 561-566.
MacLean, D. (2020) besthr - Generating Bootstrap Estimation Distributions of HR Data, Team MacLean Bioinformatics. Available at: https://github.com/TeamMacLean/besthr [Accessed August 17, 2021].
Maqbool, A., Saitoh, H., Franceschetti, M., Stevenson, C.E.M., Uemura, A., Kanzaki, H., Kamoun, S., Terauchi, R. and Banfield, M.J. (2015) Structural basis of pathogen recognition by an integrated HMA domain in a plant NLR immune receptor. eLife, 4, e08709.
Marillonnet, S., Engler, C., Klimyuk, V. and Gleba, Y. (2008) Potexvirus-derived replicon. Available at: https://patentscope.wipo.int/search/en/detail.jsf?docld=WO2008028661&tab=PCTDES CRIPTION [Accessed October 1, 2021].
Muyldermans, S. (2013) Nanobodies: natural single-domain antibodies. Annu. Rev. Biochem., 82, 775-797.
Pottinger, S.E., Bak, A., Margets, A., Helm, M., Tang, L., Casteel, C. and Innes, R.W. (2020) Optimizing the PBS1 decoy system to confer resistance to potyvirus infection in Arabidopsis and soybean. Mol. Plant-Microbe Interactions®, 33, 932-944.
Sarris, P.F., Cevik, V., Dagdas, G., Jones, J.D.G. and Krasileva, K.V. (2016) Comparative analysis of plant immune receptor architectures uncovers host proteins likely targeted by pathogens. BMC Biol., 14, 8.
Sarris, P.F., Duxbury, Z., Huh, S.U., et al. (2015) A plant immune receptor detects pathogen effectors that target WRKY transcription factors. Cell, 161, 1089-1100.
Segretin, M.E., Pais, M., Franceschetti, M., Chaparro-Garcia, A., Bos, J.I.B., Banfield, M.J. and Kamoun, S. (2014) Single amino acid mutations in the potato immune receptor R3a expand response to Phytophthora effectors. Mol. Plant-Microbe Interactions®, 27, 624-637.
Seshagiri, S. and Miller, L.K. (1997) Caenorhabditis elegans CED-4 stimulates CED-3 processing and CED-3-induced. Curr. Biol., 7, 455-460.
Signorell, A. (2021) Tools for descriptive statistics and exploratory data analysis, Available at: https://github.com/AndriSignorell/DescTools [Accessed October 1, 2021].
Tran, D.T.N., Chung, E.-H., Habring-Müller, A., Demar, M., Schwab, R., Dangl, J.L., Weigel, D. and Chae, E. (2017) Activation of a plant NLR complex through heteromeric association with an autoimmune risk variant of another NLR. Curr. Biol., 27, 1148-1160.
Wang, Jizong, Hu, M., Wang, Jia, et al. (2019) Reconstitution and structure of a plant NLR resistosome conferring immunity. Science, 364, eaav5870.
Wang, Jizong, Wang, Jia, Hu, M., et al. (2019) Ligand-triggered allosteric ADP release primes a plant NLR complex. Science, 364, eaav5868.
Wang, S., Huang, W., Duxbury, Z., Hogenhout, S.A. and Jones, J.D.G. (2021) Novel effector recognition capacity engineered into a paired NLR complex. bioRxiv, 2021.09.06.459143.
Weber, E., Engler, C., Gruetzner, R., Werner, S. and Marillonnet, S. (2011) A modular cloning system for standardized assembly of multigene constructs. PLoS ONE, 6, e16765.
Wu, C.-H., Krasileva, K.V., Banfield, M.J., Terauchi, R. and Kamoun, S. (2015) The "sensor domains" of plant NLR proteins: more than decoys? Plant-Microbe Interact., 6, 134.
Zdrzalek, R., Kamoun, S., Terauchi, R., Saitoh, H. and Banfield, M.J. (2020) The rice NLR pair Pikp-1/Pikp-2 initiates cell death through receptor cooperation rather than negative regulation. PLOS ONE, 15, e0238616.
Zhang, Z., Wang, Y., Ding, Y. and Hattori, M. (2020) Structure-based engineering of anti-GFP nanobody tandems as ultra-high-affinity reagents for purification. Sci. Rep., 10, 6239.

**Sequence Listing**

| SEQ ID NO: | description | sequence |
|---|---|---|
| 1 | pL0V-CDS1ns-Pikm-1_acceptor primer | TTGGAGTGAGACCGATATCAATACGCAAACC |
| 2 | pL0V-CDS1ns-Pikm-1_acceptor primer | TTGGAGTGAGACCGATATCAATACGCAAACC |
| 3 | pTL1 primer | TTGGTCTCAAATGGAAACCGGCAACAAG |
| 4 | pTL1 primer | TTGGTCTCAAAGCTTAGAAGCCCGGACGTTTC |
| 5 | Enhancer VHH - DNA sequence corresponding to the relevant VHH which was cloned into the vector. | TTGAAGACATAATGGCTCAGGTTCAGCTTGTTGAATCTGGTGGTGCTCTTG TTCAGCCTGGTGGTTCTTTGCGTCTTAGCTGTGCTGCTTCTGGCTTCCCTGT GAACCGTTATTCTATGCGTTGGTACCGTCAGGCTCCGGGCAAAGAACGTG AATGGGTTGCAGGTATGAGCAGCGCTGGTGATCGTAGCTCTTACGAGGAT TCTGTGAAGGGCCGTTTCACCATCTCTCGTGATGATGCTCGTAACACCGTG TACCTGCAGATGAACTCTCTGAAGCCTGAGGATACCGCTGTGTACTACTGC AATGTGAACGTGGGTTTCGAGTACTGGGGTCAGGGTACTCAGGTTACCGT TTCTTCTGGTTCGTTGTCTTCAA |
| 6 | Minimizer VHH - DNA sequence corresponding to the relevant VHH which was cloned into the vector. | TTGAAGACATAATGGCTGACGTTCAGCTTCAAGAAAGCGGTGGTGGTTCT GTTCAAGCTGGTGGATCTCTTCGTCTTAGCTGTGCTGCTTCTGGCGACACC TTCAGCTCTTATTCTATGGCCTGGTTTCGTCAGGCTCCTGGTAAAGAGTGT GAGCTGGTTAGCAACATCCTGCGTGATGGCACTACTACCTACGCTGGTTCA GTGAAGGGCCGTTTCACCATCTCACGTGATGATGCTAAGAACACCGTGTAC CTGCAGATGGTCAACCTGAAGTCTGAGGATACCGCTCGTTATTACTGCGCT GCTGATTCTGGTACTCAGCTGGGTTATGTTGGTGCTGTGGGACTTTCTTGC CTGGACTACGTTATGGATTACTGGGGTAAGGGTACTCAGGTGACCGTGTC ATCTGGTTCGTTGTCTTCAA |
| 7 | LaG_16 VHH - DNA sequence corresponding to the relevant VHH which was cloned into the vector. | TTGAAGACATAATGGCTCAGGTTCAGCTTGTTGAGTCTGGTGGTCGTCTTG TGCAGGCTGGTGATTCTCTTCGTCTTTCTTGTGCTGCTAGCGGCCGTACTTT CTCTACTTCTGCTATGGCTTGGTTTCGTCAGGCTCCTGGTCGTGAGCGTGA ATTCGTTGCTGCTATTACTTGGACCGTGGGCAACACCATTCTTGGCGATTCT GTTCGTGGCCGTTTCACCATCTCACGTGATCGTGCTCGTAACACCGTGGAC CTGCAGATGGATAATCTTGAGCCTGAGGATACCGCCGTGTACTATTGCTCT GCTCGTTCTCGTGGTTACGTGCTGTCTGTGCTTCGTTCTGTGGATAGCTATG ATTACTGGGGTCAGGGTACTCAGGTTACCGTTTCTGGTTCGTTGTCTTCAA |
| 8 | LaM_4 VHH - - DNA sequence corresponding to the relevant VHH which was cloned into the vector. | TTGAAGACATAATGGCTCAGGTTCAGCTTGTTGAGTCTGGTGGTTCTCTTG TTCAGCCTGGTGGAAGCCTTCGTCTTTCTTGTGCTGCTTCTGGCCGTTTCGC CGAGTCATCTTCTATGGGATGGTTTCGTCAGGCTCCGGGCAAAGAGCGTG AGTTCGTTGCTGCTATTTCTTGGTCAGGTGGCGCTACCAACTACGCTGATT CTGCTAAGGGACGTTTCACCCTGTCTCGTGACAATACCAAGAACACCGTGT ACCTGCAGATGAACAGCCTGAAGCCTGATGATACCGCTGTGTATTACTGCG CTGCTAACCTGGGCAACTACATCTCTTCTAACCAGCGTCTGTACGGTTACTG GGGTCAGGGTACTCAGGTTACCGTTTCTTCTCCTTTCACTGGTTCGTTGTCT TCAA |
| 9 | pJK-B2-224M primer | TTCGTCTCATCCTTCCCACACCTCCAAAACCG |
| 10 | pJK-B2-224M primer | TTCGTCTCAAGGACTACCATTGCCACAGCATTGTA |
| 11 | LaG_2 VHH - DNA sequence corresponding to the relevant VHH which was cloned into the vector. | TTGGTCTCAAATGGCTCAGGTTCAGCTTGTTGAATCTGGTGGTGGTCTTGT TCAGGCTGGTGGTTCTCTTAGACTTAGCTGTGCTGCTTCTGGCCGGACCTT CTCTAATTATGCTATGGGATGGTTCAGGCAGGCTCCTGGTAAAGAGAGAG AGTTCGTTGCTGCTATCTCTTGGACTGGCGTGTCAACCTACTACGCTGATTC TGTGAAGGGCCGTTTCACCATCAGCAGGGATAACGATAAGAACACCGTGT ACGTGCAGATGAACTCTCTGATCCCTGAGGACACCGCTATCTATTACTGCG |
| | | CTGCTGTTAGGGCCCGTAGCTTCTCTGATACTTACTCTAGGGTGAACGAGT ACGACTACTGGGGTCAGGGTACTCAGGTTACAGTTGGTTCGTGAGACCAA |
| 12 | LaG_24 VHH - DNA sequence corresponding to the relevant VHH which was cloned into the vector. | TTGGTCTCAAATGGCTGATGTGCAGCTTGTTGAATCTGGTGGTGGTCTTGT TCAGCCTGGTGGTTCTCTTAGACTTAGCTGTGCTGCTTCTGGCGAGATCGC TAGCATTATTGCTATCGGTTGGTACAGGCAGGCTCCTGGTAAGCAGAGAG AATCTGTTGCTCTGATCACCCGGTCTGGCATGATTACCTATGGTGATTCTGC TCAGGGCCGTTTCACCATCTCAAGGGATGATGCTAAGAACACCGTGTACCT GCACATGGATGATCTGGTGCCTGAAGATACCGCTGTGTACTACTGCAACG CCAAGAAGGTGAGCTTCGGAGATTATTGGGGTCAGGGTACTCAGGTTACC GTGTCTGGTTCGTGAGACCAA |
| 13 | LaM_1 VHH - DNA sequence corresponding to the relevant VHH which was cloned into the vector. | TTGGTCTCAAATGGCTCAGGTTCAGCTTGTTGAATCTGGTGGTGGTCTTGT GCAGGCTGGTGATTCTCTTAGACTTAGCTGTGCTGCTAGCGGTAGGACCTT CGAGAATTATGCTATGGGATGGTTCAGGCAGGCTCCTGGTAAAGAAAGG GAATTCGTTGGTGCTGTGTCTTGGGGTGGTGGTAGGACTTATTACGCTGA CGGTTCGTGAGACCAA |
| 14 | LaM_2 VHH - DNA sequence corresponding to the relevant VHH which was cloned into the vector. | TTGGTCTCAAATGGCTCAGGTTCAGCTTGTTGAATCTGGTGGTGGTCTTGT TCAGGCTGGTGGTTCTCTTAGACTTAGCTGCGCTACTAGCGGCTTCACCTT CTCTGATTATGCTATGGGATGGTTCAGGCAGGCTCCTGGTAAAGAGAGAG AGTTCGTTGCTGCCATCAGCTGGTCTGGTCATGTGACTGATTACGCCGATT CTGTGAAGGGCCGTTTCACCATCTCTAGGGACAATGTGAAGAACACCGTG TACCTGCAGATGAACTCTCTGAAGCCTGAGGATACCGCTGTGTATTCTTGT GCTGCTGCTAAGTCTGGCACCTGGTGGTATCAGAGGTCTGAGAACGATTT CGGTTCTTGGGGTCAGGGTACTCAGGTGACCGTTTCTAAAGAGGCTATTG GTTCGTGAGACCAA |
| 15 | LaM_3 VHH - DNA sequence corresponding to the relevant VHH which was cloned into the vector. | TTGGTCTCAAATGGCTCAGGTTCAGCTTGTTCAATCTGGTGGTGGTCTTGT TCAGGCTGGTGGATCTCTTAGACTTAGCTGTGCTGCTTCTGGCAGGACCTT CTCTGATATTGCTGTTGGTTGGTTCAGGCAGACCCCTGGTAAAGAGAGAG AGTTCGTTGCTGCCATCTCTTGGTCTGGCCTGATCATCAACTACGGCGATTC TGTTGAGGACCGGTTCACCATCTCTAGGGACAATGCTAAGTCTGCCGTGTA CCTGCAGATGAACTCTCTGAAGCCTGAGGATACCGCTGTGTATTATTGCGC TGCTAGGATCGGCATGAACTACTACTACGCTCGTGAGATCGAGTACCCTTA TTGGGGTCAGGGTACTCAGGTTACCGTGTCTAAGTGCTACGGTTCGTGAG ACCAA |
| 16 | LaM_6 VHH - DNA sequence corresponding to the relevant VHH which was cloned into the vector. | TTGGTCTCAAATGGCTCAGGTTCAGCTTGTTGAATCTGGTGGTGGTCTTGT TCAGGCTGGTGGTTCTCTTAGGCTTTCTTGTGTGGCTTCTGGCAGCGCTCCT TCTTTCTTTGCTATGGCTTGGTACAGGCAGAGCCCTGGTAATGAGAGAGAA CTGGTTGCTGCTCTTAGCAGCCTTGGCTCTACTAACTACGCTGATTCTGTGA AGGGCCGTTTCACCATCAGCATGGACAATGCTAAGAACACCGTGTACCTG CAGATGAACAACGTGAACGCTGAGGATACCGCTGTGTATTATTGCGCTGC TGGCGATTTCCACAGCTGCTACGCTAGAAAGTCTTGCGATTATTGGGGTCA GGGTACTCAGGTTACCGTGTCTGGTTCGTGAGACCAA |
| 17 | LaM_8 VHH - DNA sequence corresponding to the relevant VHH which was cloned into the vector. | TTGGTCTCAAATGGCTCAGGTTCAGCTTGTTGAATCTGGTGGTGGACTTGT TCAGGCTGGTGGATCTCTTAGGCTTTCTTGCGCTGTTTCTGGCAGGCCATT CTCTGAGTACAACCTTGGATGGTTCAGGCAGGCTCCAGGTAAAGAGAGAG AGTTCGTTGCTAGGATCAGGTCCTCTGGCACTACTGTGTACACTGATTCCG TGAAGGGCCGTTTCTCTGCTTCTAGGGATAACGCTAAGAACATGGGCTACC TCCAGCTCAACTCTCTTGAGCCAGAGGATACTGCTGTGTACTACTGCGCTA TGTCCAGGGTTGACACAGACTCTCCAGCTTTCTACGATTACTGGGGACAGG GAACTCAGGTGACAGTTTCTACTCCAAGGTCCGGTTCGTGAGACCAA |
| 18 | pICH47751_mRFP1 primer | TTGAAGACAACAGAATCATGGGTGGAATGTGAGACCGATATCAATACGCA AACC |
| 19 | pICH47751_mRFP1 primer | TTGAAGACAACATCCTCCTTCACCTGCGAATGAGACCTATAAACGCAGAAA GGC |
| 20 | EC15095 primer | TTGAAGACAAAATGGTGAGCAAGGGCGAGG |
| 21 | EC15095 primer | TTGAAGACAACATTTGAGACCACAGAGTGATTAATGAATC |
| 22 | pJK-B2-099 primer | TTCGTCTCATCCTTCCCACACCTCCAAAACCG |
| 23 | pJK-B2-099 primer | TTCGTCTCAAGGACTACCATTGCCACAGCATTGTA |
| 24 | Pikm1^Enhancer | MEAAAMAVTAATGALAPVLVKLAALLDDGECNLLEGSRSDAEFIRSELEAVHS LLTPNILGRMGDDDAACKDGLIAEVRELSYDLDDAVDDFLELNFEQRRSASPF GELKARVEERVSNRFSDWKLPAASLPPSSVHRRAGLPPPDAGLVGMHKRKEE LIELLEQGSSDASRWRKRKPHVPLRIMGGMAQVQLVESGGALVQPGGSLRLS CAASGFPVNRYSMRWYRQAPGKEREWVAGMSSAGDRSSYEDSVKGRFTISR DDARNTVYLQMNSLKPEDTAVYYCNVNVGFEYWGQGTQVTVSSGSQVKED VKEITAMLAPVKSICEFHEVKTICILGLPGGGKTTIARVLYHALGTQFQCRVFASI SPSSSPSPNLTETLADIFAQAQLGVTDTLSTPYGGSGTGRALQQHLIDNISAFLL NKKYLIVIDDIWHWEEWEVIRKSIPKNDLGGRIIMTTRLNSIAEKCHTDDNDVF VYEVGDLDNNDAWSLSWGIATKSGAGNRIGTGEDNSCYDIVNMCYGMPLA LIWLSSALVGEIEELGGAEVKKCRDLRHIEDGILDIPSLQPLAESLCLGYNHLPLY LRTLLLYCSAYHWSNRIERGRLVRRWIAEGFVSEEKEAEGYFGELINRGWITQH GDNNSYNYYEIHPVMLAFLRCKSKEYNFLTCLGLGSDTSTSASSPRLIRRLSLQG GYPVDCLSSMSMDVSHTCSLVVLGDVARPKGIPFYMFKRLRVLDLEDNKDIQ DSHLQGICEQLSLRVRYLGLKGTRIRKLPQEMRKLKHLEILYVGSTRISELPQEIG ELKHLRILDVRNTDITELPLQIRELQHLHTLDVRNTPISELPPQVGKLQNLKIMC VRSTGVRELPKEIGELNHLQTLDVRNTRVRELPWQAGQISQSLRVLAGDSGD GVRLPEGVCEALINGIPGATRAKCREVLSIAIIDRFGPPLVGIFKVPGSHMRIPK MIKDHFRVLSCLDIRLCHKLEDDDQKFLAEMPNLQTLVLRFEALPRQPITINGT GFQMLESFRVDSRVPRIAFHEDAMPNLKLLEFKFYAGPASNDAIGITNLKSLQK VVFRCSPWYKSDAPGISATIDVVKKEAEEHPNRPITLLINAGYKEISTESHGSSE NIAGSSGIDTEPAQAQHDNLPAVRDDYKGKGILLDGRCPTCGRATKIEEETQD RVADIEIQTETTS |
| 25 | Pikm1^LaG16 | MEAAAMAVTAATGALAPVLVKLAALLDDGECNLLEGSRSDAEFIRSELEAVHS LLTPNILGRMGDDDAACKDGLIAEVRELSYDLDDAVDDFLELNFEQRRSASPF GELKARVEERVSNRFSDWKLPAASLPPSSVHRRAGLPPPDAGLVGMHKRKEE LIELLEQGSSDASRWRKRKPHVPLRIMGGMAQVQLVESGGRLVQAGDSLRLS CAASGRTFSTSAMAWFRQAPGREREFVAAITWTVGNTILGDSVRGRFTISRD RARNTVDLQMDNLEPEDTAVYYCSARSRGYVLSVLRSVDSYDYWGQGTQVT VSGSQVKEDVKEITAMLAPVKSICEFHEVKTICILGLPGGGKTTIARVLYHALGT QFQCRVFASISPSSSPSPNLTETLADIFAQAQLGVTDTLSTPYGGSGTGRALQQ HLIDNISAFLLNKKYLIVIDDIWHWEEWEVIRKSIPKNDLGGRIIMTTRLNSIAEK CHTDDNDVFVYEVGDLDNNDAWSLSWGIATKSGAGNRIGTGEDNSCYDIVN MCYGMPLALIWLSSALVGEIEELGGAEVKKCRDLRHIEDGILDIPSLQPLAESLC LGYNHLPLYLRTLLLYCSAYHWSNRIERGRLVRRWIAEGFVSEEKEAEGYFGELI NRGWITQHGDNNSYNYYEIHPVMLAFLRCKSKEYNFLTCLGLGSDTSTSASSP RLIRRLSLQGGYPVDCLSSMSMDVSHTCSLVVLGDVARPKGIPFYMFKRLRVL DLEDNKDIQDSHLQGICEQLSLRVRYLGLKGTRIRKLPQEMRKLKHLEILYVGST RISELPQEIGELKHLRILDVRNTDITELPLQIRELQHLHTLDVRNTPISELPPQVGK LQNLKIMCVRSTGVRELPKEIGELNHLQTLDVRNTRVRELPWQAGQISQSLRV LAGDSGDGVRLPEGVCEALINGIPGATRAKCREVLSIAIIDRFGPPLVGIFKVPG SHMRIPKMIKDHFRVLSCLDIRLCHKLEDDDQKFLAEMPNLQTLVLRFEALPR QPITINGTGFQMLESFRVDSRVPRIAFHEDAMPNLKLLEFKFYAGPASNDAIGI TNLKSLQKVVFRCSPWYKSDAPGISATIDVVKKEAEEHPNRPITLLINAGYKEIS TESHGSSENIAGSSGIDTEPAQAQHDNLPAVRDDYKGKGILLDGRCPTCGRAT KIEEETQDRVADIEIQTETTS |
| 26 | Pikm1^LaM4 | MEAAAMAVTAATGALAPVLVKLAALLDDGECNLLEGSRSDAEFIRSELEAVHS LLTPNILGRMGDDDAACKDGLIAEVRELSYDLDDAVDDFLELNFEQRRSASPF GELKARVEERVSNRFSDWKLPAASLPPSSVHRRAGLPPPDAGLVGMHKRKEE LIELLEQGSSDASRWRKRKPHVPLRIMGGMAQVQLVESGGSLVQPGGSLRLS CAASGRFAESSSMGWFRQAPGKEREFVAAISWSGGATNYADSAKGRFTLSR DNTKNTVYLQMNSLKPDDTAVYYCAANLGNYISSNQRLYGYWGQGTQVTVS |
| | | SPFTGSQVKEDVKEITAMLAPVKSICEFHEVKTICILGLPGGGKTTIARVLYHAL GTQFQCRVFASISPSSSPSPNLTETLADIFAQAQLGVTDTLSTPYGGSGTGRAL QQHLIDNISAFLLNKKYLIVIDDIWHWEEWEVIRKSIPKNDLGGRIIMTTRLNSI AEKCHTDDNDVFVYEVGDLDNNDAWSLSWGIATKSGAGNRIGTGEDNSCY DIVNMCYGMPLALIWLSSALVGEIEELGGAEVKKCRDLRHIEDGILDIPSLQPLA ESLCLGYNHLPLYLRTLLLYCSAYHWSNRIERGRLVRRWIAEGFVSEEKEAEGYF GELINRGWITQHGDNNSYNYYEIHPVMLAFLRCKSKEYNFLTCLGLGSDTSTS ASSPRLIRRLSLQGGYPVDCLSSMSMDVSHTCSLVVLGDVARPKGIPFYMFKR LRVLDLEDNKDIQDSHLQGICEQLSLRVRYLGLKGTRIRKLPQEMRKLKHLEILY VGSTRISELPQEIGELKHLRILDVRNTDITELPLQIRELQHLHTLDVRNTPISELPP QVGKLQNLKIMCVRSTGVRELPKEIGELNHLQTLDVRNTRVRELPWQAGQIS QSLRVLAGDSGDGVRLPEGVCEALINGIPGATRAKCREVLSIAIIDRFGPPLVGI FKVPGSHMRIPKMIKDHFRVLSCLDIRLCHKLEDDDQKFLAEMPNLQTLVLRF EALPRQPITINGTGFQMLESFRVDSRVPRIAFHEDAMPNLKLLEFKFYAGPASN DAIGITNLKSLQKVVFRCSPWYKSDAPGISATIDVVKKEAEEHPNRPITLLINAG YKEISTESHGSSENIAGSSGIDTEPAQAQHDNLPAVRDDYKGKGILLDGRCPTC GRATKIEEETQDRVADIEIQTETTS |
| 27 | Pik-m1 | MEAAAMAVTAATGALAPVLVKLAALLDDGECNLLEGSRSDAEFIRSELEAVHS LLTPNILGRMGDDDAACKDGLIAEVRELSYDLDDAVDDFLELNFEQRRSASPF GELKARVEERVSNRFSDWKLPAASLPPSSVHRRAGLPPPDAGLVGMHKRKEE LIELLEQGSSDASRWRKRKPHVPLRIMGGEMQKIVFKIPMVDDKSRTKAMSL VASTVGVHSVAIAGDLRDQVVVVGDGIDSINLVSALRKKVGPAMFLEVSQVK EDVKEITAMLAPVKSICEFHEVKTICILGLPGGGKTTIARVLYHALGTQFQCRVF ASISPSSSPSPNLTETLADIFAQAQLGVTDTLSTPYGGSGTGRALQQHLIDNISA FLLNKKYLIVIDDIWHWEEWEVIRKSIPKNDLGGRIIMTTRLNSIAEKCHTDDN DVFVYEVGDLDNNDAWSLSWGIATKSGAGNRIGTGEDNSCYDIVNMCYGM PLALIWLSSALVGEIEELGGAEVKKCRDLRHIEDGILDIPSLQPLAESLCLGYNHL PLYLRTLLLYCSAYHWSNRIERGRLVRRWIAEGFVSEEKEAEGYFGELINRGWI TQHGDNNSYNYYEIHPVMLAFLRCKSKEYNFLTCLGLGSDTSTSASSPRLIRRLS LQGGYPVDCLSSMSMDVSHTCSLVVLGDVARPKGIPFYMFKRLRVLDLEDNK DIQDSHLQGICEQLSLRVRYLGLKGTRIRKLPQEMRKLKHLEILYVGSTRISELPQ EIGELKHLRILDVRNTDITELPLQIRELQHLHTLDVRNTPISELPPQVGKLQNLKI MCVRSTGVRELPKEIGELNHLQTLDVRNTRVRELPWQAGQISQSLRVLAGDS GDGVRLPEGVCEALINGIPGATRAKCREVLSIAIIDRFGPPLVGIFKVPGSHMRI PKMIKDHFRVLSCLDIRLCHKLEDDDQKFLAEMPNLQTLVLRFEALPRQPITIN GTGFQMLESFRVDSRVPRIAFHEDAMPNLKLLEFKFYAGPASNDAIGITNLKSL QKVVFRCSPWYKSDAPGISATIDVVKKEAEEHPNRPITLLINAGYKEISTESHGS SENIAGSSGIDTEPAQAQHDNLPAVRDDYKGKGILLDGRCPTCGRATKIEEET QDRVADIEIQTETTS |
| 28 | Pik-m1_-_CC | EAAAMAVTAATGALAPVLVKLAALLDDGECNLLEGSRSDAEFIRSELEAVHSLL TPNILGRMGDDDAACKDGLIAEVRELSYDLDDAVDDFLELNFEQRRSASPFGE LKARVEERVSNRFSDWKLPAASLPPSSVHRRAGLPPPDAGLVGMHKRKEELIE LLEQGSSDASRWRKRKPHVPLRI |
| 29 | Pik-m1_-LRR | TCLGLGSDTSTSASSPRLIRRLSLQGGYPVDCLSSMSMDVSHTCSLVVLGDVA RPKGIPFYMFKRLRVLDLEDNKDIQDSHLQGICEQLSLRVRYLGLKGTRIRKLPQ EMRKLKHLEILYVGSTRISELPQEIGELKHLRILDVRNTDITELPLQIRELQHLHTL DVRNTPISELPPQVGKLQNLKIMCVRSTGVRELPKEIGELNHLQTLDVRNTRV RELPWQAGQISQSLRVLAGDSGDGVRLPEGVCEALINGIPGATRAKCREVLSI AIIDRFGPPLVGIFKVPGSHMRIPKMIKDHFRVLSCLDIRLCHKLEDDDQKFLAE MPNLQTLVLRFEALPRQPITINGTGFQMLESFRVDSRVPRIAFHEDAMPNLKL LEFKFYAGPASNDAIGITNLKSLQKVVFRCSPWYKSDAPGISATIDVVKKEAEE HPNRPITLLINAGYKEISTESHGSSENIAGSSGIDTEPAQAQHDNLPAVRDDYK GKGILLDGRCPTCGRATKIEEETQDRVADIEIQTETTS |
| 30 | Pik-m1_-_NB-ARC | KEDVKEITAMLAPVKSICEFHEVKTICILGLPGGGKTTIARVLYHALGTQFQCRV FASISPSSSPSPNLTETLADIFAQAQLGVTDTLSTPYGGSGTGRALQQHLIDNIS AFLLNKKYLIVIDDIWHWEEWEVIRKSIPKNDLGGRIIMTTRLNSIAEKCHTDD NDVFVYEVGDLDNNDAWSLSWGIATKSGAGNRIGTGEDNSCYDIVNMCYG MPLALIWLSSALVGEIEELGGAEVKKCRDLRHIEDGILDIPSLQPLAESLCLGYN HLPLYLRTLLLYCSAYHWSNRIERGRLVRRWIAEGFVSEEKEAEGYFGELINRG WITQHGDNNSYNYYEIHPVMLAFLRCKSKEYNFL |
| 31 | Pik-m2 | MELVVGASEATMKSLLGKLGNLLAQEYALISGIRGDIQYINDELASMQAFLRD LSNVPEGHSHGHRMKDWMKQIRDIAYDVEDCIDDFAHRLPQDSISDAKWSF LLTKIYELWTWWPRRVIASNIAQLKVRAQQIADRRSRYGVNNPEHLDSSSSAR TRAVNYEIAEYQVTSPQIIGIKEPVGMKTVMEELEVWLTNPQAENGQAVLSIV GFGGVGKTTIATALYRKVSEKFQCRASVAVSQNYDQGKVLNSILSQVSNQEQ GSSTTISEKKNLTSGAKSMLKTALSLLRGNCICQPENDGNPDNTPIRLQETTDD DQNPRKLEQLLAEKSYILLIDDIWSAETWESIRSILPKNNKGGRIIVTTRFQAVG STCSPLETDRLHTVDFLTDDESQNLFNTSICESKIRKDSNKVDEQVPEEIWKICG GLPLAIVSMAGLVACNPRKACCDWSKLCKSLFPEQETPLTLDGVTRILDCCYN DLPADLKTCLLYLSIFPKGWKISRKRLSRRWIAEGFANEKQGLTQERVAEAYFN QLTRRNLVRPMEHGSNGKVKTFQVHDMVLEYIMSKSIEENFITVVGGHWQ MTAPSNKVRRLSMQSSGSNRGSSTKGLNLAQVRSLTVFGNLNHVPFHSFNY GIIQVLDLEDWKGLKERHMTEICQMLLLKYLSIRRTEISKIPSKIQKLEYLETLDIR ETYVRDLPKSIVQLKRIISILGGNKNTRKGLRLPQEKSKKPIKNPSPQGKTKEPAK KGFLSQEKGKGAMKALRVLSGIEIVEESSEVAAGLHQLTGLRKLAIYKLNITKGG DTFKQLQSSIEYLGSCGLQTLAINDENSEFINSLGDMPAPPRYLVALELSGKLEK LPKWITSITTLNKLTISVTVLRTETLEILHILPSLFSLTFAFSLSAAKQDQDIIKDILE NNKLDSDGEIVIPAEGFKSLKLLRFFAPLVPKLSFLDKNAMPALEIIEMRFKDFE GLFGIEILENLREVHLKVSDGAEAITKFLVNDLKVNTEKPKVFVDGIVTA |

| | | |
|---|---|---|
| Note on the sequences. SEQ ID NOs: 1 to 4, 9-10, 18-23 are primer pairs used for cloning. SEQ ID NOs: 5 to 8, 11-17 are VHH (nanobody) sequences used in the examples. SEQ ID NOs: 24 to 26 are full length sequences of engineered Pik-1 pikobodies. SEQ ID NO: 27 is the full length native Pik-1 sequence. SEQ ID NOs: 28, 29, and 30 are, respectively, sequences of the Pik-1 CC, LRR, and NB domains. SEQ ID NO: 31 is the full length native Pik-2 sequence. | | |

## Claims

1. A chimeric protein comprising a binding molecule, preferably a nanobody, linked to a plant immune receptor protein.

2. A chimeric protein according to claim 1, wherein the nanobody is specific for an effector protein from a plant pathogen, and the chimeric protein is capable of activating immune signalling in a plant cell upon binding of the effector protein to the nanobody.

3. A chimeric protein according to claim 1 or 2, wherein the plant immune receptor protein is an NLR protein.

4. A chimeric protein according to claim 3, wherein the NLR protein has the general structure CC-NB-LRR.

5. A chimeric protein according to claim 3 or 4, wherein the nanobody is integrated internally within the NLR protein.

6. A chimeric protein according to any of claims 3 to 5, wherein the NLR protein is derived from a wild-type NLR protein which comprises an integrated domain, and, in the modified NLR protein, the nanobody partially or completely replaces the integrated domain.

7. A chimeric protein according to claim 6, wherein the integrated domain of the wild-type NLR protein is a HMA domain or a AvrRpt cleavage/NOI domain, preferably a HMA domain.

8. A chimeric protein according to any one of claims 3 to 7, wherein the NLR protein corresponds to Pik-1, RGA5 or Pii-2, preferably Pik-1.

9. A chimeric protein according to any one of the preceding claims, wherein the nanobody is a camelid nanobody.

10. A chimeric protein according to any one of the preceding claims, wherein the plant immune receptor is linked to two or more nanobodies, preferably wherein each nanobody is specific for a different pathogen effector protein.

11. A chimeric protein according to claim 1 or 2, wherein the plant immune receptor is a receptor kinase or a receptor like protein.

12. A nucleic acid molecule comprising a nucleotide sequence encoding a chimeric protein as defined in any preceding claim.

13. A vector comprising a nucleic acid molecule as defined in claim 12.

14. A plant cell comprising a chimeric protein as defined in any one of claims 1-11, a nucleic acid molecule as defined in claim 12 or a vector as defined in claim 13.

15. A plant or plant part comprising a plant cell as defined in claim 14.

16. A method of producing a modified plant cell, plant part or plant, wherein the method comprises introducing into a plant cell, or at least one plant cell of a plant part or plant, a chimeric protein as defined in any one of claims 1-11, a nucleic acid molecule as defined in claim 12 or a vector as defined in claim 13.

17. A method of enhancing immunity of a plant against a pathogen, the method comprising providing a plant with a chimeric protein as defined in any one of claims 1-11, a nucleic acid molecule as defined in claim 12 or a vector as defined in claim 13.

18. A plant protection product comprising a chimeric protein as defined in any one of claims 1-11, a nucleic acid molecule as defined in claim 12 or a vector as defined in claim 13.
